# EUROPEAN PATENT APPLICATION

(11) **EP 1 852 113 A2**
(43) Date of publication of application: **07.11.2007**
(21) Application number: 07000173.0
(22) Date of filing: 07.12.2001
(51) Int. Cl.: A61K 31/13, A61P 25/18, A61P 25/22, A61P 25/24

(54) **Methods for treating neuropsychiatric disorders with NMDA receptors antagonists**

(30) Priority: 07.12.2000 US 254007 P
(62) Divisional of application: 01990191.7
(71) Applicant: Neuromolecular Inc., La Jolla, CA 92037 (US)
(72) Inventor: Lipton, Stuart M.D., Rancho Santa Fe, CA 92067-4018 (US); Went, Gregory T., Mill Valley, CA 9494-1204 (US)
(74) Representative: Aston, Heidi Frances

(57) **Abstract**

The Present invention relates to compositions and methods for treating a human patient afflicted with a neuropsychiatric disorder. Specifically, the invention provides for compositions and methods of modulating or antagonizing the activity of neuronal NMDA receptors, wherein such antagonistic activity is capable of modulating the glutamate induced excitatory response of the neurons, thereby inhibiting an excitotoxic effect, promoting a neurotrophic effect, and thereby providing a therapeutic effect that treats the neuropsychiatric disorder.

## Description

### FIELD OF THE INVENTION

The present invention relates to compositions and methods for treating a human patient having an affliction comprising a neuropsychiatric disorder. Specifically, the invention provides for compositions and methods of modulating or antagonizing the activity of neuronal ionotropic glutamate receptors, such as NMDA receptors, wherein such antagonistic activity is capable of modulating the excitatory response of the neurons, inhibiting an excitotoxic effect, and promoting a neurotrophic effect, thereby providing a therapeutic effect that treats the neuropsychiatric disorder.

### BACKGROUND OF THE INVENTION

Receptors to the neuroexcitatory amino acid, glutamate, particularly the N-methyl-D-aspartate (NMDA) subtype of these receptors, play critical roles in the development, function and death of neurons (see, Mc Donald J W et al., Brain Research Reviews, 15: 41-70 (1990) and Choi W, Neuron, 1: 623-34 (1988) incorporated herein by reference). The N-methyl-D-aspartate (NMDA) receptor is a postsynaptic, ionotropic receptor which is responsive to, *inter alia,* the excitatory amino acids glutamate and glycine and the synthetic compound NMDA, hence the receptor name. The NMDA receptor controls the flow of both divalent (Ca²⁺) and monovalent (Na⁺ and K⁺) ions into the postsynaptic neural cell through a receptor associated channel (*see,* Foster et al., Nature, 329: 395-396 (1987); Mayer et al., Trends in Pharmacol. Sci., 11:254-260 (1990) incorporated herein by reference).

The NMDA receptor has been implicated during development in specifying neuronal architecture and synaptic connectivity, and may be involved in experience dependent synaptic modifications, In addition, NMDA receptors are also thought to be involved in long term potentiation, central nervous system (CNS) plasticity, cognitive processes, memory acquisition, retention, and learning. Furthermore, the NMDA receptor has also drawn particular interest since it appears to be involved in a broad spectrum of CNS disorders. For instance, during brain ischemia caused by stroke or traumatic injury, excessive amounts of the excitatory amino acid glutamate are released from damaged or oxygen deprived neurons. This excess glutamate binds to the NMDA receptor which opens the ligand-gated ion channel thereby allowing Ca²⁺ influx producing a high level of intracellular Ca²⁺ which activates biochemical cascades resulting in protein, DNA, and membrane degradation leading to cell death. This phenomenon, known as excitotoxicity, is also thought to be responsible for the neurological damage associated with other disorders ranging from hypoglycemia and cardiac arrest to epilepsy. In addition, there are preliminary reports indicating similar involvement in the chronic neurodegeneration of Huntington's, Parkinson's, and Alzheimer's diseases. Activation of the NMDA receptor has been shown to be responsible for post-stroke convulsions, and, in certain models of epilepsy, activation of the NMDA receptor has been shown to be necessary for the generation of seizures. Blockage of the NMDA receptor Ca²⁺ channel by the animal anesthetic PCP (phencyclidine) produces a psychotic state in humans similar to schizophrenia (reviewed in Johnson et al., Annu. Rev. Pharmacol. Toxicol., 30: 707-750 (1990) incorporated herein by reference). Further, NMDA receptors have also been implicated in certain types of spatial learning, (*see*, Bliss et al., Nature, 361: 31 (1993), incorporated herein by reference). Interestingly, both the spatial and temporal distribution of NMDA receptors in mammalian nervous systems have been found to vary. Thus, cells may produce NMDA receptors at different times in their life cycles and not all neural cells may utilize the NMDA receptor.

Due to its broad-spectrum of neurological involvement, yet non-universal distribution, investigators have been interested in the identification and development of drugs capable of acting on the NMDA receptor. Drugs that can modulate the NMDA receptor are expected to have enormous therapeutic potential. For instance, U.S. Pat. No. 4,904,681, issued to Cordi et al., and incorporated herein by reference, describes the use of D-cycloserine, which was known to modulate the NMDA receptor, to improve and enhance memory and to treat cognitive deficits linked to a neurological disorder. D-cycloserine is described as a glycine agonist which binds to the strychnine-insensitive glycine receptor.

U.S. Pat. No. 5,061,721, issued to Cordi et al., and incorporated herein by reference, describes the use of a combination of D-cycloserine and D-alanine to treat Alzheimer's disease, age-associated memory impairment, learning deficits, and psychotic disorders, as well as to improve memory or learning in healthy individuals. D-alanine is administered in combination with D-cycloserine to reduce the side effects observed in clinical trials of D-cycloserine, mainly those due to its growth-inhibiting effect on bacteria resulting in depletion of natural intestinal flora. D-Alanine reverses the growth-inhibiting effect of D-cycloserine on bacteria. It is also reported that D-cycloserine actually has partial agonist character.

U.S. Pat. No. 5,086,072, issued to Trullas et al.*,* and incorporated herein by reference, describes the use of 1-aminocyclopropanecarboxylic acid (ACPC), which was known to modulate the NMDA receptor as a partial agonist of the strychnine-insensitive glycine binding site, to treat mood disorders including major depression, bipolar disorder, dysthymia and seasonal effective disorder. It is also therein described that ACPC mimics the actions of clinically effective antidepressants in animal models. In addition, a copending U.S. patent application is cited that describes that ACPC and its derivatives may be used to treat neuropharmacological disorders resulting from excessive activation of the NMDA receptor. However, there remains a need in the art for a satisfactory method of modulating NMDA receptor function.

Development of drugs targeting the NMDA receptor, although desirous, has been hindered because the structure of the NMDA receptor has not yet been completely elucidated. It is believed to consist of several protein chains (subunits) embedded in the postsynaptic membrane. The first two subunits determined so far form a large extracellular region which probably contains most of the allosteric binding sites, several transmembrane regions looped and folded to form a pore or channel which is permeable to Ca²⁺ and a carboxyl terminal region with an as yet unknown function. The opening and closing of the channel is regulated by the binding of various ligands to domains of the protein residing on the extracellular surface and separate from the channel As such, these ligands are all known as allosteric ligands. The binding of two co-agonist ligands (glycine and glutamate) is thought to effect a conformational change in the overall structure of the protein which is ultimately reflected in the channel opening, partially open, partially closed, or closed. The binding of other allosteric ligands modulates the conformational change caused or effected by glutamate and glycine. It is believed that the channel is in constant motion, alternating between a cation passing (open) and a cation blocking (closed) state. It is not known at present whether the allosteric modulators actually increase the time during which the channel is open to the flow of ions, or whether the modulators increase the frequency of opening. Both effects might be occurring at the same time.

Several compounds are known which are antagonistic to the flow of cations through the NMDA receptor but which do not competitively inhibit the binding of allosteric ligands to any of the known sites. Instead, these compounds bind inside the open cation channel and are generally known as channel blockers. In fact, binding of a tritiated form of one such channel blocker, dizocilpine (*i*.*e*., MK-801), is a good measure of the activation of the NMDA receptor complex. When the channel is open, MK-801 may freely pass into the channel and bind to its recognition site in the channel. Conversely, when the channel is closed, MK-801 may not freely pass into the channel and bind. When the channel is partially closed, less MK-801 is able to bind than when the channel is fully open,

Channel blockers such as MK-801 and antagonists are known to protect cells from excitotoxic death but, in their case, the cure may be as undesirable as the death since they block any flux of Ca²⁺ thereby eliminating any chance of resumed normal activity. Channel blockers and glutamate site antagonists are known to cause hallucinations, high blood pressure, loss of coordination, vacuolation in the brain, learning disability and memory loss. PCP, a typical channel blocker, produces a well characterized schizophrenic state in man.

Other divalent cations such as Mg²⁺ and Zn²⁺ can modulate the NMDA receptor. The exact location of the divalent cation binding site(s) is still unclear. Zn²⁺ appears to be antagonistic to channel opening and appears to bind to an extracellular domain. Mg²⁺ shows a biphasic activation curve - at low concentrations it is an agonist for NMDA receptor function, and at high concentrations it is a receptor antagonist. It appears to be absolutely necessary for proper receptor functioning and appears to bind at two sites - a voltage dependant binding site for Mg²⁺ within the channel and another non-voltage dependent binding site on the extracellular domain. These compounds can modulate the NMDA receptor but are not appropriate for long term therapy. There is a need in the art for a safe and effective compound for treating neuropsychiatric disorders.

Recurrent mood disorders can have devastating long-term effects, and the cost of these illnesses in terms of human suffering, productivity and health care is enormous. It is now recognized that, for many patients, the long-term outcome is often much less favorable than previously thought, with incomplete interepisode recovery, and a progressive decline in overall functioning observed (Goldberg and Harrow, 1996; Tohen *et al.,* 2000). Indeed, according to the Global Burden of Disease Study, mood disorders are among the leading causes of disability worldwide, and are likely to represent an increasingly greater health, societal, and economic problem in the coming years (Murray and Lopez, 1997). Many antidepressants are currently available for the treatment of acute depression. Until a few decades ago, tricyclic antidepressants (TCAs) were the only drugs available for the treatment of depression. Monoamine oxidase inhibitors (MAOIs) were available and now, are seldom used. Then came a number of new drugs in rapid succession of new drugs, among them the selective serotonin reuptake inhibitors (SSRIs) which are now widely used. Although options for pharmacologic treatment for depression have grown seemingly exponentially over the past several decades, the current armamentarium of antidepressants continues to have limitations of both efficacy and tolerability. Thus, there is a clear need to develop novel and improved therapeutics for major depression.

### SUMMARY OF THE INVENTION

The present invention provides a method for treating neuropsychiatric disorders comprising administering to a human patient suffering from a neuropsychiatric disorder, an effective amount of an NMDA receptor antagonist compound, wherein the compound modulates glutamatergic neurotransmission by the receptor, thereby treating or alleviating the neuropsychiatric disorder and thereby providing a therapeutic effect. In one aspect the compound provides robust neurotrophic effects via direct intracellular mechanisms. In another aspect, excessive glutamatergic transmission is modulated, thereby mediating the excitotoxic effect of glutamate on neurons and thereby providing a neuroprotective effect. In another aspect, the NMDA receptor antagonist compound modulates glutamatergic activation of the cortico-striatal or subthallamicopalladial pathways.

In one embodiment, the neuropsychiatric disorder is major depressive disorder. In another embodiment, the neuropsychiatric disorder is bipolar disorder, In yet another embodiment, the neuropsychiatric disorder is anxiety. In still another embodiment, the neuropsychiatric disorder is a drag-related disorder such as drug addiction, drug dependency, drug withdrawal, or drug tolerance.

The invention provides for uses of NMDA receptor antagonists to treat patients with major depression without psychotic features according to the DSM-N criteria, as well as methods of improving overall depressive symptomatology, by administering to the patient a therapeutically effective dosage of the compound. In one embodiment, the compound is memantine. In another embodiment, the compound is a nitromemantine derivative,

The invention also provides for methods of assessing the neurotrophic effects of NMDA receptor antagonist compounds in the treatment of patients with neuropsychiatric disorders and methods of determining whether compound-induced alterations in brain glutamate (Glu) levels are associated with responsiveness to the compound's therapeutic effects. In one embodiment, the invention likewise provides for methods of assessing the effects of memantine or nitromemantine derivatives on glucose metabolism in unipolar depression.

The present invention provides for the use of NMDA receptor antagonist compounds that are formulated into medicaments used in the treatment of patients suffering from neuropsychiatric disorders. The NMDA receptor antagonist compounds are of the following formula or pharmaceutically acceptable salts thereof:

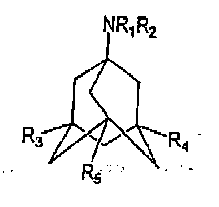

The groups R₁, R₂, R₃, R₄ and R₅ of the formula are independently denned. R₁ is H, alkyl, heteroalkyl, aryl, heteroaryl, C(O)O R₆ or C(O)R₆. R₂ is H, alkyl, heteroalkyl, aryl, heteroaryl, C(O)OR₆ or C(O)R₆. R₃ is H, alkyl, heteroalkyl, aryl or heteroaryl. R₄ is H, alkyl, heteroalkyl, aryl or heteroaryl. R₅ is OR₇, alkyl-OR₇ or heteroalkyl-OR₇. R₆ is alkyl, heteroalkyl, aryl or heteroaryl. R₇ is NO₂, C(O)R₆, C(O)alkyl-ONO₂ or C(O)heteroalkyl-ONO₂. The following substituents are preferred : R₁ and R₂ are H; R₃ and R₄ are H or alkyl; and, R₇ is NO₂ or C(O)alkyl-ONO₂.

The present invention also provides pharmaceutical compositions that can be used to treat a neurological disorder. The compositions include a pharmaceutically acceptable carrier and one or more compounds of the following formula or pharmaceutically acceptable salts thereof:

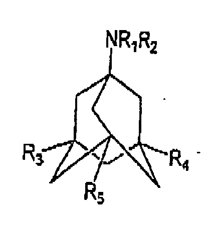

The substituents of the compounds are independently defined. R₁ is H, alkyl, hetexoalkyl, aryl, heteroaryl, C(O)OR₆ or C(O)R_{6.} R₂ is H, alkyl, heteroalkyl aryl, heteroaryl, C(O)OR₆ or C(O)R₆. R₃ is H, alkyl, heteroalkyl, aryl or heteroaryl, R₄ is H, alkyl, heteroalkyl, aryl or heteroaryl, R₅ is OR₇, alkyl-OR7 or heteroalkyl-OR₇. R₆ is alkyl, heteroalkyl, aryl or heteroaryl. R₇ is NO₂, C(O)R₆, C(O)alkyl-ONO₂ or C(O)heteroalkyl-ONO₂. The following substituents are preferred: R₁ and R₂ are H; R₃ and R₄ are H or alkyl; and, R₇ is NO₂ or C(O)alkyl-ONO₂.

The present invention also provides methods of treating a neurological disorder. The methods include administering to a patient a pharmaceutically acceptable carrier and one or more compounds of the following formula, or pharmaceutically acceptable salts thereof:

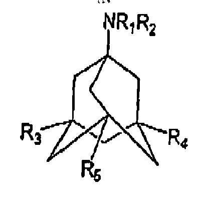

The substituents of the compounds are independently defined. R₁ is H, alkyl, heteroalkyl, aryl, heteroaryl, C(O)OR₆ or C(O)R₆. R₂ is H, alkyl, heteroalkyl, aryl, heteroaryl, C(O)OR₆ or C(O)R₆. R₃ is H, alkyl, heteroalkyl, aryl or heteroaryl R₄ is H, alkyl, heteroalkyl, aryl or heteroaryl. R₅ is OR₇, alkyl-OR₇ or heteroalkyl-OR₇. R₆ is alkyl, heteroalkyl, aryl or heteroaryL R₇ is NO₂, C(O)R₆, C(O)alkyl-ONO₂ or C(O)heteroalky-ONO₂. The following substituents are preferred: R₁ and R₂ are H; R₃ and R₄ are H or alkyl; and, R₇ is NO₂ or C(O)alkyl-ONO₂.

The present invention further provides methods of making medicaments comprising NMDA receptor antagonist compounds of the following formula or pharmaceutically acceptable salts thereof:

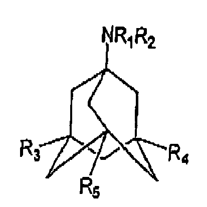

The substituents of the compounds are independently defined. R₁ is H, alkyl, heteroalkyl, aryl, heteroaryl, C(O)OR₆ or C(O)R₆. R₂ is H, alkyl, heteroalkyl, aryl, heteroaryl, C(O)OR₆ or C(O)R₆. R₃ is H, alkyl, heteroalkyl, aryl or heteroaryl. R₄ is H, alkyl, heteroalkyl, aryl or heteroaryl. R₅ is OR₇, alkyl-OR₇ or heteroalkyl-OR₇, R₆ is alkyl, heteroalkyl, aryl or heteroaryl. R₇ is NO₂, C(O)R₆, C(O)alkyl-ONO₂ or C(O)heteroalkyl-ONO₂. The following substituents are preferred: R₁ and R₂ are H; R₃ and R₄ are H or alkyl; and, R₇ is NO₂ or C(O)alkyl-ONO₂. Preferably, the methods involve oxidizing a compound of the following formula;

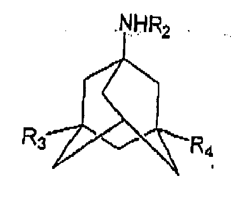

Preferably, the methods further involve nitrating a compound of the formula:

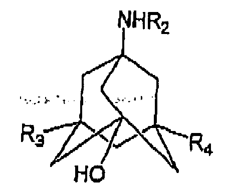

Preferably, the compound is treated with H₂SO₄ and water in the oxidation step. The nitration step preferably includes treatment with HNO₃ and Ac₂O.

### BRIEF DESCRIPTION OF THE DRAWINGS

FIG. 1 shows the synthesis of an adamantane nitrate derivative.
FIG. 2 shows the synthesis of an adamantane ester derivative.
FIG. 3 shows the synthesis of halo and nitrate substituted adamantane ester derivatives.
FIG. 4 shows the synthesis of an alkyl-ONO₂ derivative of adamantane.
FIG. 5 shows the inhibition of NMDA induced apoptosis in cerebrocortical neurons by compound 7, Cerebrocortical cultures were exposed to 300 µM NMDA for 20 mm with or without various concentrations of compound 7. The next day cultures were analyzed by neuronal apoptosis as described in Example 19. Neuronal apoptosis was largely prevented by compound 7 in a dose-dependent manner (P < 0.001, n =3 cultures in each case).
FIG. 6 shows that administration of compound 7 decreases cerebral damage after stroke in a murine cerebral ischemia model as compared to both a control and memantine (see Example 20). Use of the intraluminal suture method demonstrated (n = 3 for each group) that compound 7 was effective in decreasing cerebral damage after stroke (P < 0.03 from control: P <0.05 from memantine).
FIG. 7 shows that administration of compound 8 relaxes a precontracted aortic vessel in a dose-dependent fashion (see Example 21). FIG. 7a shows that relaxations were seen at 10⁻⁶M and complete relaxation was achieved at 10.6 M. FIG. 7b shows the effect of solvent. FIG. 7c shows that relaxations were attenuated by methylene blue. FIG. 7d shows that relaxations were attenuated by hemoglobin.
FIG. 8 shows that the action of aminoadamantane derivatives are specific. Compound 9 (a) and 10 (c) produced either no effect or slight blood vessel contractions that were comparable to those produced by solvent (EtOH) alone. Compound 7 (b) produced modest relaxation at a 10 µM concentration.
FIG. 9 illustrates studies with memantine, indicative of its activating or antidepressant properties.

### DETAILED DESCRIPTION OF THE INVENTION

The present invention provides for compositions and methods of treating neuropsychiatric disorders by modulating the activity of NMDA subtype glutamate receptors in patients afflicted with one or more neuropsychiatric disorders. The compounds used in the present invention modulate glutamatergic neurotransmission and provide or exert robust neurotrophic effects via direct intracellular mechanisms, thereby treating or alleviating the neuropsychiatric disorder.

As used herein, a "neuropsychiatric disorder" refers to acute and subacute disorders with both neurological and psychiatric features, Examples of common neuropsychiatric disorders that are treatable by the present invention comprise major depressive disorder (MDD), bipolar disorder (manic-depressive illness or BPD), anxiety, and drug addiction including dependence, withdrawal, and drug tolerance, disorders arising from trauma, ischemic or hypoxic conditions including stroke, hypoglycemia, cerebral ischemia, cardiac arrest, spinal cord trauma, head trauma, perinatal hypoxia, cardiac arrest and hypoglycemic neuronal damage, epilepsy, Alzheimer's disease, Huntington's disease, Parkinsonism, amyotrophic lateral sclerosis, convulsion, pain, schizophrenia, muscle spasms, migraine headaches, urinary incontinence, emesis, brain edema, tardive dyskinesia, AIDS-induced dementia, ocular damage, retinopathy, cognitive disorders, and neuronal injury associated with HIV-infection such as dysfunction in cognition, movement and sensation. Neuropsychiatric disorders are described in Diagnostic and Statistical Manual of Mental Disorders, 4^{th} Ed, American Psychiatric Press, (1994) incorporated herein by reference.

As used herein, an "NMDA receptor antagonist compound" refers to aminoadamantane derivatives such as memantine, nitromemantine compounds, and related memantine and nitromemantine derivatives which use memantine as a NMDAR channel blocker and a nitric oxide species to regulate the redox modulatory site on the NMDA receptor. Such NMDA receptor antagonist compounds are specified in U.S. patent nos. 6,071,876, 5,801,203, 5,747,545, 5,614,5 60, 5,506,231, and PCT application 01/62706, all to Lipton, S.A., et al., and incorporated herein by reference.

As used herein, the term "Alkyl" refers to unsubstituted or substituted linear, branched or cyclic alkyl carbon chains of up to 15 carbon atoms. Linear alkyl groups include, for example, methyl, ethyl, N-propyb N-butyl, N-pentyl, N-hoxyl, N-heptyl and N-octyl. Branched alkyl groups include, for example, iso-propyl, sec-butyl, iso-butyl, tert-butyl and neopentyl. Cyclic alkyl groups include, for example, cyclopropyl, cyclobutyl, cyclopentyl and cyclohexyl. Alkyl groups can be substituted with one or more substituents. Nonlimiting examples of such substituents include NO₂, ONO₂, F, Cl, Br, I, OH, OCR₃, CO₂H, CO₂CH₃, CN, aryl and heteroaryl. Where "alkyl" is used in a context such as "alkyl-ONO₂," it refers to an alkyl group that is substituted with a ONO₂ moiety. Where "alkyl" is used in a context such as "C(O)alkyl-ONO₂," it refers to an alkyl group that is connected to a carbonyl group at one position and that is substituted with a ONO₂ moiety.

As used herein, the term "Heteroalkyl" refers to unsubstituted or substituted linear, branched or cyclic chains of up to carbon atoms that contain at least one heteroatom (e.g., nitrogen, oxygen or sulfur) in the chain. Linear heteroalkyl groups include, for example, CH₂CH₂OCH₃, CH₂CH₂N(CH₃)₂ and CH₂CH₂SCH₃. Branched groups include, for example, CH₂CH(OCH₃)CH₃, CH₂CH(N(CH₃)₂)CH₃ and CH₂CH(OCH₃)CH₃. Cyclic beteroalkyl groups include, for example, CH(CH₂CH₂)₂O, H(CH₂CH₂)₂NCH and CH(CH₂CH₂)₂S. Heteroalkyl groups can be substituted with one or more substituents. Nonlimiting examples of such substituents include NO₂, ONO₂, F, Cl, Br, I, OH, OCR₃, CO₂H, CO₂CH₃, CN, aryl and heteroaryl. Where "heteroalkyl" is used in a context such as "heteroalkyl-ONO₂," it refers to a heteroalkyl group that is substituted with an ONO₂ moiety. Where "heteroalkyl" is used in a context such as "C(O)heteroalkyl- NO₂," it refers to an alkyl group that is connected to a carbonyl group at one position and that is substituted with a ONO₂ moiety.

As used herein, the term "Halo" refers to F, Cl, Br or 1.

As used herein, the term "Aryl" refers to an unsubstituted or substituted aromatic, carbocyclic group. Aryl groups are either single ring or multiple condensed ring compounds. A phenyl group, for example, is a single ring, aryl group. An aryl group with multiple condensed rings is exemplified by a naphthyl group. Aryl groups can be substituted with one or more substituents. Nonlimiting examples of such substituents include NO₂, ONO₂, F, Cl, Br, I, OH, OCR₃, CO₂H, CO₂CH₃, CN, aryl and heteroaryl.

As used herein, the term "Heteroaryl" refers an unsubstituted or substituted aromatic group having at least one heteroatom (e.g., nitrogen, oxygen or sulfur) in the aromatic ring. Heteroaryl groups are either single ring or multiple condensed ring compounds. Single ring heteroaryl groups having at least one nitrogen include, for example, tetrazoyl, pyrrolyl, pyridyl, pyridazinyl, indolyl, quinolyl, imidazolyl, isoquinolyl, pyrazolyl, pyrazinyl, pyrimidinyl and pyridazinonyl. A furyl group, for example is a single ring heteroaryl group containing one oxygen atom. A condensed ring heteroaryl group containing one oxygen atom is exemplified by a benzofuranyl group. Thienyl, for example, is a single ring heteroaryl group containing one sulfur atom. A condensed ring heteroaryl group containing one sulfur atom is exemplified by benzothienyl heteroaryl groups containing more than one kind of heteroatom in the same ring. Examples of such groups include furazanyl, oxazolyl, isoxazolyl, thiazolyl and phenothiazinyl. Heteroaryl groups can be substituted with one or more substituents. Nonlimiting examples of such substituents include NO₂, ONO₂, F, Cl, Br, I, OH, OCH₃, CO₂H, CO₂CH₃, CN, aryl and heteroaryl.

As used herein, a "therapeutic effect" refers to an observable improvement over the baseline clinically observable signs and symptoms of a neuropsychiatric disorder, as measured by the techniques disclosed herein.

The term "pharmaceutically acceptable" refers to a lack of unacceptable toxicity in a compound, such as a salt or excipient. Pharmaceutically acceptable salts include inorganic anions such as chloride, bromide, iodide, sulfate, sulfite, nitrate, nitrite, phosphate, and the like, and organic anions such as acetate, malonate, pyruvate, propionate, cinnamate, tosylate, citrate, and the like. Pharmaceutically acceptable excipients are described at length by E. W. Martin, in Remington's Pharmaceutical Sciences (Mack Pub. Co.).

As used herein, "direct intracellular mechanisms" refer to effects on intracellular signaling pathways that either promote neuroprotection or block cell death (apoptotic) and injury pathways.

As used herein, "glutamatergic neurotransmission" refers to synaptic transmission between nerve cells in the brain whereby glutamate is released from the presynaptic cell onto the postsynaptic cell to bind to glutamate receptors, thereby triggering an electrical current in the postsynaptic cell. This process effects information transfer between the nerve cells. In the cases referred to here, the type of glutamate receptor on the postsynaptic cell that is most implicated in the pathophysiology of the neuropsychiatric manifestations is the NMDA subtype of glutamate receptor.

As used herein, "providing a neurotrophic effect" refers to the upregulation of intracellular signaling pathways in response to NMDA receptor antagonists, that enhance neuronal survival and are generally regulated by neurotrophic factors such as brain-derived neurotrophic factor (BDNF).

As used herein, "decreasing the pathophysiology of depressive disorders" refers to a decrease in an underlying event for depression that consists of overstimulation of glutamate receptors, especially of the NMDA receptor subtype.

As used herein, "excessive glutamate-induced currents" refers to overstimulation of glutamate receptors, leading to excessive Ca²⁺ influx, free radical formation and other biochemical events that contribute to nerve cell toxicity, damage, and even cell death (due to either necrosis or apoptosis).

As used herein, "substantially without dopamine or norepinephrine" refers to concentrations of these neurotransmitters insufficient to trigger and propagate an action potential in the postsynaptic cell.

Neuropsychiatric mood disorders such as major depressive disorder (MDD) and bipolar disorder (manic-depressive illness, BPD) are common, severe, chronic and often life-threatening illnesses. Suicide is estimated to be the cause of death in up to approximately 15 percent of individuals afflicted with MDD, and in addition to suicide, many other deleterious health-related effects are increasingly being recognized (see, Musselman *et al.,* 1998; Schulz *et al.,* 2000, incorporated herein by reference). Far from being diseases with purely psychological manifestations, MDD are systemic diseases with deleterious effects on multiple organ systems. For example, MDD represent a major risk factor for both the development of cardiovascular disease, as well as for death after an index myocardial infarction. Furthermore, a recent study, which controlled for physical illness, smoking and alcohol consumption, found that the magnitude of the increased mortality risk conferred by the presence of high depressive symptoms was similar to that of stroke and congestive heart failure. The cumulative effects of recurring bouts of affective episodes lead to an increased rate of marital and family breakdown, unemployment, impaired career progress and consequent financial difficulties. The costs associated with disability and premature death represents an economic burden of tens of billions of dollars annually in the United States alone (Greenberg *et al*., 1990; Wyatt and Henter, 1995, incorporated herein by reference). It is thus not altogether surprising that the Global Burden of Disease Study has identified MDD among the leading causes of disability worldwide, and as illnesses which are likely to represent an increasingly greater health, societal, and economic problem in the coming years (Murray and Lopez, 1997, incorporated herein by reference). Suicide is the cause of death in 10-20% of individuals afflicted with either bipolar or recurrent depressive disorders. Despite the devastating impact MDD's have on the lives of millions worldwide, little is known about their etiology or pathophysiology. Furthermore, despite the availability of a wide range of antidepressant drugs, clinical trials indicate that 30% to 40% of depressed patients fail to respond to first-line antidepressant treatment despite adequate dosage, duration, and compliance (Nierenberg, 1994; Thase and Rush, 1995, incorporated herein by reference).

Morphometric neuroimaging studies have demonstrated that, *in toto,* patients with both BPD and MDD display morphometric changes suggestive of cell loss and/or atrophy (Drevets *et al.,* 1997; Drevets, 1999; Sheline *et al.,* 1996; 1999, incorporated herein by reference), Volumetric neuroimaging studies show an enlargement of third and lateral ventricles, as well as reduced gray matter volumes in the orbital and medial prefrontal cortex (PFC), the ventral striatum, and the mesiotemporal cortex in patients with mood disorders (Drevets, 1999; Sheline et *al*., 1996; 1999). Reductions in frontal lobe volumes, and striking ~40% reductions in the mean gray matter volume in the region located ventral to the genu of the corpus callosum have recently been demonstrated in BPD depressives and familial unipolar depressives (Drevets *et al.,* 1997). Reductions in the volume of the hippocampus also have been observed in subjects with a history of MDD findings, which may persist for decades after the depressive episodes have resolved (Bremner *et al.,* 2000, incorporated herein by reference; Sheline *et al.,* 1996; Sheline *et al.,* 1999). Loss of hippocampal volume appears to be correlated with the total lifetime duration of MDD (Sheline *et al.,* 1999), leading to the suggestion that these changes may represent the sequellae of repeated and/or prolonged episodes of depression (Brown *et al*., 1999; Sapolsky, 2000).

A number of studies have now shown that initial abnormally low brain N-acetyl aspartate (NAA) measures may increase and even normalize with remission of CNS symptoms in disorders such as demyelinating disease, amyotrophic lateral sclerosis (ALS), mitochondrial encephalopathies, and HIV-associated dementia (Tsai and Coyle, 1995). NAA is now generally regarded as a measure of neuronal viability and function, rather than strictly as a marker for neuronal loss *per se* (Tsai and Coyle, 1995). In recent studies using high-resolution magnetic resonance spectroscopic imaging methods, Bertolino et al. (1999) found decreased NAA levels bilaterally in the hippocampus of BPD subjects compared to controls. Decreased levels of NAA also have been found bilaterally in the dorsolateral prefrontal cortex (DLPFC) in BPD patients, compared to healthy controls (Winsberg *et al.,* 2000). These studies add neurochemical support to the contention that mood disorders are associated with regional neuronal loss and/or reductions in neuronal viability/function, illustrating both a physical and a psychological dimension to the disorder. In addition to the accumulating neuroimaging evidence, several postmortem brain studies are now providing direct evidence for reductions in regional CNS volume, cell number and cell body size. Baumann and associates (1999) reported reduced volumes of the left nucleus accumbens, the right putamen and bilateral pallidum externum in postmortem brain samples obtained from patients with unipolar MDD or BPD, Several recent postmortem stereological studies of the PFC also have demonstrated reduced regional volume, cell numbers and/or sizes. Morphometric analysis of the density and size of cortical neurons in the DLPFC and orbitofrontal cortices has revealed significant reductions in mood disorders patients as compared to control subjects (Rajkowska *et al*., 1999; 2000). Overall, the preponderance of the data from the neuroimaging studies and the growing body of postmortem evidence presents a convincing case that there is indeed a reduction in regional CNS volume, accompanied by atrophy and in some cases loss of cells (both neurons and glia) in at least a subset of patients with mood disorders.

### Influence of Antidepressant Treatment on Cell Survival Pathways

Factors involved in neuronal atrophy and survival are targets of antidepressant treatments in the present invention. Important pathways involved in cell survival and plasticity that contribute to providing a neurotrophic effect include, for example, the cAMP-CREB cascade, as well as a CREB target, brain derived neurotrophic factor (BDNF). These can be up-regulated by antidepressant treatment (Duman *et al.,* 2000, incorporated herein by reference). Upregulation of CREB and BDNF occurs in response to several different classes of antidepressant treatments, including norepinephrine (NE) and SSRIs and electroconvulsive seizure, indicating that the cAMP-CREB cascade and BDNF are common post-receptor targets of therapeutic compounds (Nibuya *et al.,* 1995, 19%). In addition, upregulation of CREB and BDNF is dependent on chronic treatment, consistent with the therapeutic action of antidepressants. Upregulation of the cAMP-CREB cascade and BDNF increases performance in behavioral models of depression (Duman *et al.,* 2000). Antidepressant treatments produce neurotrophic-like effects, such as a greater regeneration of catecholamine axon terminals in the cerebral cortex (Nakamura, 1990). Use of NMDA receptor antagonist compounds to modulate the excess activity of the glutamatergic system provides a neurotrophic effect to the patient thereby decreasing the pathophysiology of this neuropsychiatric disorder.

### Antidepressant Activity of NMDA Antagonists and Other Drugs that Affect Glutamate Neurotransmission

The monoamine hypothesis of depression, which was developed for the pharmacological effects of early drug development, no longer provides a satisfactory explanation of the mode of action of all antidepressant compounds or of the underlying pathophysiology in depression. In the 1950s, D-cycloserine, a partial agonist at the NMDA receptor glycine site used as a part of multi-drug anti-tuberculosis treatment, was reported to have a mood elevating effects (Heresco-Levy and Javitt, 1998). Since then, there is increasing evidence for an association between alterations of brain glutamatergic neurotransmission and the pathophysiology of mood disorders. A growing body of preclinical research suggests that the NMDA class of glutamate receptors may be involved in the pathophysiology of major depression and the mechanism of action of antidepressants (Skolnick *et al.,* 1999). NMDA receptor antagonists such as MK-801 and AP-7, have demonstrated antidepressant effects in animal models of depression, including the application of inescapable stressors, forced-swim, and tail suspension-induced immobility tests, in learned helplessness models of depression, and in animals exposed to a chronic mild stress procedure (Hauang, 1997; Paul, 1997). Conversely, antidepressant administration has been shown to affect NMDA receptor function (Nowak et al, 1993, 1995) and receptor binding profiles (Paul *et al*., 1994). Furthermore, the role of glutamatergic dysfunction in depression is further supported by the fact that repeated antidepressant administration regionally alters expression of mRNA that encodes multiple NMDA receptor subunits (Boyer *et al*., 1998; Skolnick, 1999) and radioligand binding to these receptors within circumscribed areas of the central nervous system (CNS) (Skolnick, 1999). In summary, behavioral and neurochemical studies suggest that NMDA antagonists produce neurochemical alterations in the brain similar to antidepressant drugs and that they show an antidepressant-like behavioral profile in some animal models of depression. A growing body of preclinical evidence suggests that existing antidepressants, upon chronic administration, exert significant dampening (albeit complex) effects on the glutamatergic system. Furthermore, many stress paradigms are believed to exert many of their deleterious effects on hippocampal structures via enhancement of glutamatergic neurotransmission. Overall, modulation of the excess activity of the glutamatergic system provides a method of treating the pathophysiology of neuropsychiatric disorders. More specifically, compositions and methods that dampen glutamatergic activity provide a method of administering a therapeutic antidepressant effect to a patient afflicted with or suffering from a depressive disorder.

### NMDA Receptor Antagonists

Given the potential role of glutamate in CNS injury and neurodegenerative diseases, several treatment strategies have been implemented to reduce glutamate-mediated excitotoxicity. One approach involves the use of NMDA receptor antagonist compounds, Ketamine has been studied in depression but is associated with an increased risk of developing psychosis. Also, psychomimetic effects were reported to occur with other NMDA antagonists. Lamotrigine in a double-blind, placebo-controlled study was reported to be effective in acute bipolar depression (Calabrese *et al.,* 1999). In unipolar depression, lamotrigine was found to be superior to placebo in last observation carried forward HAMD item 1 and CGI severity change but not in total score HAMD and MADRS (Laurenza et *al*., 1999). Although the exact mechanism of action of lamotrigine is unknown, an inhibition of an excessive release of glutamate is postulated as a likely mechanism of action for this drug (Calabrese et *al.*, 1999). It is possible that drugs that preferentially affect glutamatergic neurotransmission may be effective in certain subgroups of patients with depression. Further studies are required to precisely define whether certain subgroups of patients with depression (unipolar and bipolar depression) are likely to respond differently to anti-glutamatergic compounds, One such compound that reduces the release of glutamate and has been shown to be neuroprotective in animal models of Parkinson' disease, dementia, ischemia, and traumatic CNS injury, is the NMDA receptor antagonist memantine, which unlike other glutamate receptor antagonists, appears to spare normal neurotransmission and blocks only excessive glutamate-induced currents, as demonstrated in patch-clamp electrophysiological recordings correlated to behavioral studies (*see*, Chen *et al*., 1992, 1988, Chen and Lipton, 1997, Lipton, 1993, Lipton and Rosenberg 1994, incorporated herein by reference).

Memantine (Akatinol Memantine®, (Merz & Co., GmbH) CAS Registry No. 41100-52-1), is an uncompetitive *N*-methyl-D-aspartate (NMDA) antagonist currently used for the treatment of dementia syndrome, spinal spasticity and Parkinson's disease. Chemically, memantine is 1-amino-3,5-dimethyladamantane of the adamantine class. Compared to the other NMDA antagonists, memantine has been reported to have the greatest effective potency for binding at the PCP and MK-801 receptor sites in human brain tissue (Kornhuber *et al*., 1991). Memantine binds to the PCP and MK-801 binding sites of the NMDA receptor in postmortem human frontal cortex at therapeutic concentrations (Kornhuber *et al.,* 1989), and reduces membrane currents (Bormann, 1989). Memantine is well tolerated, and despite its wide use in Germany, only a few isolated cases of psychosis and cognitive deficits have been reported with its use. Compared to other NMDA antagonists, memantine appears to have a more favorable pharmacological profile and is less likely to induce psychosis and cognitive deficits. Without being bound by theory, one possibility why memantine is less likely to induce cognitive deficits and psychosis may be due its negligible effects on the hypothalamic-pituitary axis (HPA) compared to other NMDA antagonists such as ketamine. NMDA receptors have been reported to be involved in the physiologic pulsatile regulation of hormone release from the HPA axis (Bhat *et al.,* 1995) resulting in hypercortisolemia. Psychotic symptoms and cognitive deficits in depression has been linked to an increased dopamine activity secondary to this HPA overactivity (Walder *et al*., 2000). The lack of memantine's effect on the HPA axis and resulting increased dopamine activity maybe an explanation for the low rates of psychosis seen with this drug. Another advantage of menantine over other NMDA antagonists is that contrary to, for example, dextromethorphan, memantine has no active metabolites that possess NMDA. antagonizing properties (Ziemann *et al*., 1996). Furthermore, memantine serum levels are available for measurement. Memantine is one of the few NMDA antagonists available for use in humans and is ideal for treating major depression as it and its precursors amantadine, have been in clinical use for many years with minimal side-effects (Kornhuber *et al.,* 1994). Rarely has memantine been associated with significant the side-effects of agitation, confusion, and psychosis (Rabey *et al.,* 1992; Riederer *et al.,* 1991) as seen with other NMDA antagonists, such as phencyclidine and ketamine. Memantine is well tolerated in the geriatric populations for which it is typically prescribed in Europe (Gortelmeyer *et al.,* 1992).

Memantine has significant neurotrophic and activating properties, and it can be used to modulate glutamatergic neurotransmission, while also providing for robust neurotrophic effects *via* direct intracellular mechanisms. Memantine displays potent non-competitive voltage-dependent NMDA antagonist properties with effects comparable to MK-801 (see, Bormann, 1989, incorporated herein by reference). Memantine also demonstrates anticonvulsant and neuroprotective properties and dopaminergic effects *in vitro* (*see,* Maj, 1982, incorporated herein by reference). Memantine has been used since 1978 and is approved in Germany for the treatment of mild and moderate cerebral performance disorders with the following cardinal symptoms: concentration and memory disorders, loss of interest and drive, premature fatigue, and dementia syndrome, as well as in diseases in which an increase of attention and alertness (vigilance) is required. Cerebral and spinal spasticity, Parkinson and Parkinson-like diseases are other indications. Memantine acts as a modulator of glutamatergic neurotransmission. In the states of a reduced glutamate release, after degeneration of neurons, memantine results in an improvement in signal transmission and activation of neurons, In the state of a massive glutamate release, e.g., ischemia, memantine blocks NMDA receptors that mediate the excitotoxic action of glutamate on neurons. It is believed that its neuroprotective properties are due to NMDA receptor antagonism in pathologies with increased glutamate. Memantine's efficacy in Parkinson's Disease has been suggested to be a result of its ability to neutralize (or modulate) the increased activity of the glutamatergic cortico-striatal and subthalamicopallidal pathways (Klockgether and Turski, 1989, 1990, and Schmidt *et al.,* 1990, incorporated herein by reference). This effect is independent of dopamine or norepinephrine release.

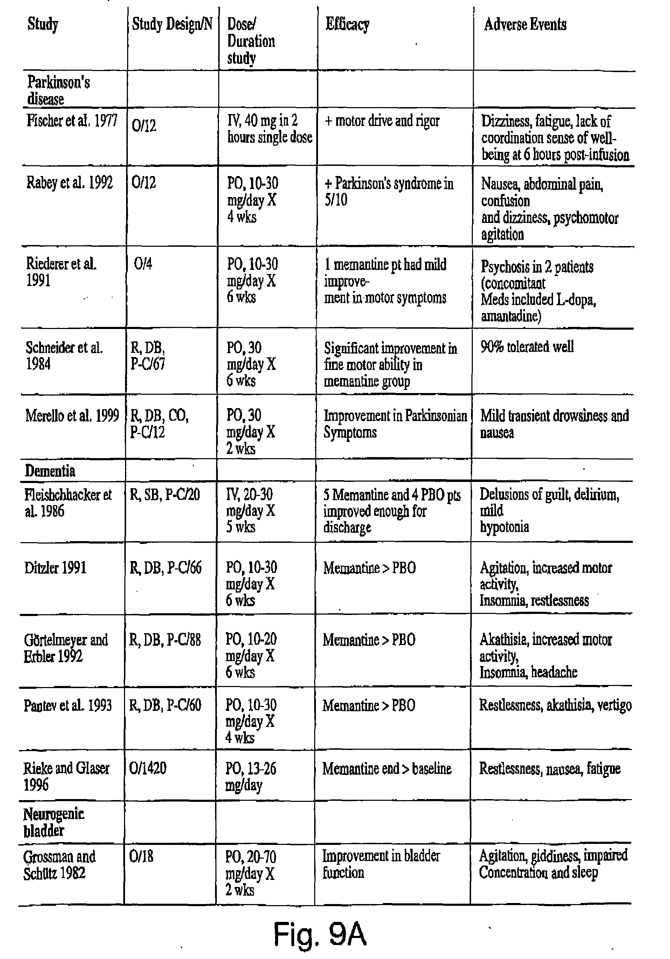

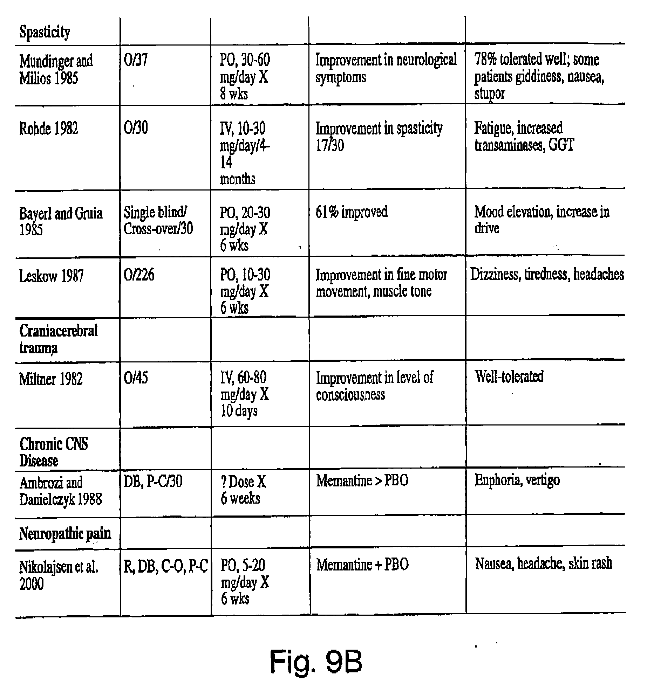

Memantine has been reported for many years to have positive effects on deficit symptoms or depressive symptoms commonly found in other neuropsychiatric disorders such as Parkinson's disease and dementia. In studies of patients with dementia and Parkinson's disease, the symptoms of depressed mood, anxiety, lack of drive, somatic disturbances, impairment in vigilance, short-term memory and concentration were significantly improved with memantine. Some of these studies also reported the adverse events of hyperactivity, restlessness, and euphoria with memantine, suggesting that it may have activating or antidepressant properties. These findings are summarized in the table shown in FIG. 9.

### Pharmacology of Memantine

Memantine is quickly and completely absorbed and is practically unbound to human albumin (<10%). Its elimination is biphasic. The average half-life of memantine is reported to be 4-9 hours for the first therapeutically relevant phase, and then 40-65 hours for the second phase. Elimination occurs primarily by the renal route in 75%-90%, and fecal excretion is only about 10%-25% (Weseman *et al.,* 1980). Side effects of memantine are dose dependent and include dizziness, internal and motoric restlessness and agitation, fatigue, congestion in the head, and nausea, Some isolated cases of confusion and psychosis have been reported but these patients also had concomitant medical illnesses and were also receiving L-dopa or amantadine (Rabey *et al.,* 1992; Ditzler, 1991). An increase in motor activity and euphoria or giddiness has also been reported to occur with memantine treatment. The drug interactions with memantine in general are mild and have been reported to occur with barbiturates, neuroleptics, anticholinergics, L-dopa, dopaminergic agonists and amantadine.

The NMDA receptor antagonist compounds of the present invention comprise aminoadamantane derivatives that can be formulated into medicaments comprising pharmaceutically acceptable salts or in a pharmaceutical composition further comprising excipients. Memantine and nitromemantine derivatives are administered to human patients across dosage ranges from 0,1 to 1000 mg/day. The currently preferred therapeutic dose of memantine is approximately 5-35 mg/day, but memantine is well tolerated at doses of 100-500 mg/day. Dosages of nitromemantine compounds are commonly 1-100 mg/day, and are similarly tolerated. The serum levels of memantine in humans have been reported to range between 0.25 and 0.529 µM at a dose between 5 and 30 mg/day. In these same patients, the CSF levels of memantine ranged between 0.122 and 0.053 µM and are highly correlated to serum values (r = 0.99, p = 0.0018). The mean CSF/serum ratio was 0.52 (see, Kornhuber and Quack, 1995, incorporated herein by reference). At these concentrations (0.1 to 1000 mg/day), memantine specifically interacts with the PCP or MK-108 binding site of the NMDA receptor (Kornhuber *et al*., 1994; Kornhuber and Quack, 1995), and is effective at modulating glutamatergic neurotransmission by the receptor.

The NMDA receptor antagonist compounds that are administered in a pharmaceutical composition are mixed with a suitable carrier or excipient such that a therapeutically effective amount is present in the composition. The term "therapeutically effective amount" refers to an amount of the compound that is necessary to achieve a desired endpoint (e.g., decreasing neuronal damage as the result of a stroke). As such, a therapeutic endpoint in a dosage regimen is recognized by the development of a therapeutic effect in the patient, as determined by the assessments and techniques disclosed herein. A medical professional can determine the appropriate dosage regimen for memantine, and adjust the patient's dose upward or downward as needed to provide a therapeutic effect and minimize adverse side effects. It will be understood, however, that the specific dose level for any particular patient will depend upon a variety of factors including the activity of the specific compound employed, the age, body weight, general health, sex, diet, time of administration, route of administration and rate of excretion, drug combination and the severity of the particular disease undergoing therapy.

The NMDA receptor antagonist compound, its enantiomers or a pharmaceutically acceptable salt thereof (the active compound) may be administered orally, topically, parenterally, intranasally by inhalation or spray, or rectally in dosage unit formulations containing conventional non-toxic pharmaceutically acceptable carriers, adjuvants and vehicles. The amount of active compound that may be combined with the carrier materials to produce a single dosage form will vary depending upon the host treated and the particular mode of administration. The term parenteral as used herein includes subcutaneous injections, intravenous, intramuscular, intrasternal injection or infusion techniques. In addition, the invention provides a pharmaceutical formulation comprising a NMDA receptor antagonist compound and a pharmaceutically acceptable carrier. The active compound may be present in association with one or more non-toxic pharmaceutically acceptable carriers and/or diluents and/or adjuvants and if desired other active ingredients. The pharmaceutical compositions containing the active compound may be in a form suitable for oral use, for example, as tablets, troches, lozenges, aqueous or oily suspensions, dispersible powders or granules, emulsion, hard or soft capsules, or syrups or elixirs.

Compositions intended for oral use may be prepared according to any method known to the art for the manufacture of pharmaceutical compositions and such compositions may contain one or more compounds selected from the group consisting of sweetening compounds, flavoring compounds, coloring compounds and preserving compounds in order to provide pharmaceutically elegant and palatable preparations. Tablets contain the active compound in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating compounds, for example, corn starch, or alginic acid; binding compounds, for example starch, gelatin or acacia, and lubricating compounds, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active material in admixture with excipients suitable for the manufacture of aqueous suspensions. Such excipients are suspending compounds, for example sodium carboxymethylcellulose, methylcellulose, hydropropylmethylcellulose, sodium alginate, polyvinylpyrrolidone, gum tragacanth and gum acacia; dispersing or wetting compounds may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring compounds, one or more flavoring compounds, and one or more sweetening compounds, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening compound, for example beeswax, hard paraffin or acetyl alcohol Sweetening compounds such as those set forth above, and flavoring compounds may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting compound, suspending compound and one or more preservatives. Suitable dispersing or wetting compounds and suspending compounds are exemplified by those already mentioned above. Additional excipients, for example sweetening, flavoring and coloring compounds, may also be present.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying compounds may be naturally occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation products of the said partial esters with ethylene oxide, for example sweetening, flavoring and coloring compounds, may also be present,

Syrups and elixirs may be formulated with sweetening compounds, for example glycerol, propylene glycol, sorbitol or sucrose, Such formulations may also contain a demulcent, a preservative and flavoring and coloring compounds. The pharmaceutical compositions may be in the form of a sterile injectable aqueous or oleaginous suspension. This suspension may be formulated according to the known art using those suitable dispersing or wetting compounds and suspending compounds which have been mentioned above. The sterile injectable preparation may also be sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables,

The active compound may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

The active compound may be administered parenterally in a sterile medium. The drug, depending on the vehicle and concentration used can either be suspended or dissolved in the vehicle. Advantageously, adjuvants such as local anesthetics, preservatives and buffering compounds can be dissolved in the vehicle.

The compounds of the present invention are NMDA receptor antagonists comprising aminoadamantane derivatives such as memantine, nitromemantine, and the like. The NMDA receptor antagonists are of the following formula:

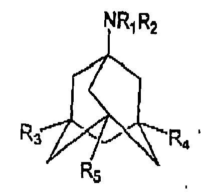

The groups R₁, R₂, R₃, R₄and R₅ of the formula are independently defined. R₁ is H, alkyl, heteroalkyl, aryl, heteroaryl, C(O)OR₆or C(O)R₆. R₂ is H, alkyl, heteroalkyl, aryl, heteroaryl, C(O)OR₆ or C(O)R₆. R₃ is H, alkyl, heteroalkyl, aryl or heteroaryl. R₄ is H, alkyl, heteroalkyl, aryl or heteroaryl. R₅ is OR₇, alkyl-OR7 or heteroalkyl-OR₇. R₆ is alkyl, heteroalkyl, aryl or heteroaryl. R₇ is NO₂, C(O)R₆, C(O)alkyl]-ONO₂ or C(O)heteroalkyl-ONO₂. The following substituents are preferred: R₁ and R₂ are H; R₃ and R₄ are H or alkyl; and, R₇ is NO₂ or C(O)alkyl-ONO₂.

Preferably, R₁ is H and R₂ is H, C(O)O-alkyl or C(O)O-aryl, Where R₂ is C(O)O-alkyl, it is preferred that the alkyl group is methyl, ethyl, N-propyl, iso-propyl, N-butyl, sec-butyl, tert-butyl or benzyl Where R₂ is C(O)O-aryl, it is preferred that the aryl group is phenyl or a substituted phenyl. More preferably, R₁ and R₂ are both H.

Preferably, both R₃ and R₄ are H or linear alkyl groups. Where R₃ and R₄ are both alkyl groups, it is preferred that the groups are methyl, ethyl, N-propyl, N-butyl, see-butyl, tert-butyl or benzyl.

Preferably, R₅ is ONO₂, O-alkyl-ONO2 or OC(O)-alkyl-ONO₂, Where R₅ is O-alkyl-ONO₂, it is preferred that the alkyl group be CH₂, CH₂CH₂ or CH₂CH₂CH₂. Where R₅ is OC(O)-alkyl-ONO₂, it is preferred that the alkyl group be CH₂, CH₂CH₂, CH₂CH₂CH₂ or CH₂ CH₂CH₂CH₂. More preferably, R₅ is ONO₂.

The NMDA receptor antagonist compounds of the present invention are synthesized starting from a haloadamantane derivative. The haloadamantane derivative is treated with acid and a nitrile to form an amidoadamantane derivative. Treatment of the NMDA receptor antagonist compounds with an acid and second reagent provides a functionalized NMDA receptor antagonist compound.

In certain cases, the second reagent used to form the functionalized NMDA receptor antagonist compound is water. The compound formed in this case is an amido alcohol. The amido alcohol is either nitrated to provide an amido nitrate derivative or hydrolyzed to provide an amino alcohol derivative. Where an amino alcohol is formed, a variety of different steps can be used to make other NMDA receptor antagonist compounds, including the following nonlimiting examples: 1) protection of the amine group, followed by nitration of the alcohol group and deprotection of the amine group to provide an amino nitrate derivative; 2) protection of the amine group, followed by esterification of the alcohol group and deprotection of the amine group to provide an amino ester derivative; and, 3) protection of the amine group, followed by esterification to with a halogenated acid chloride and nucleophilic displacement to provide an carbamate nitrate-ester derivative.

In other cases, the second reagent used to form the functionalized NMDA receptor antagonist compound is formic acid. The compound formed in this case is an amido acid. The amido acid is subjected to conditions that form an amido alkanol. The amido alkanol is either nitrated to provide an amido alkane nitrate derivative or deprotected to provide an amino alkanol derivative. Where an amino alkanol derivative is formed, the amine group is protected to form an amido alkanol derivative, which is subsequently nitrated to provide an amido alkane-nitrate derivative. Deprotection of the amido group affords an amino alkane-nitrate derivative.

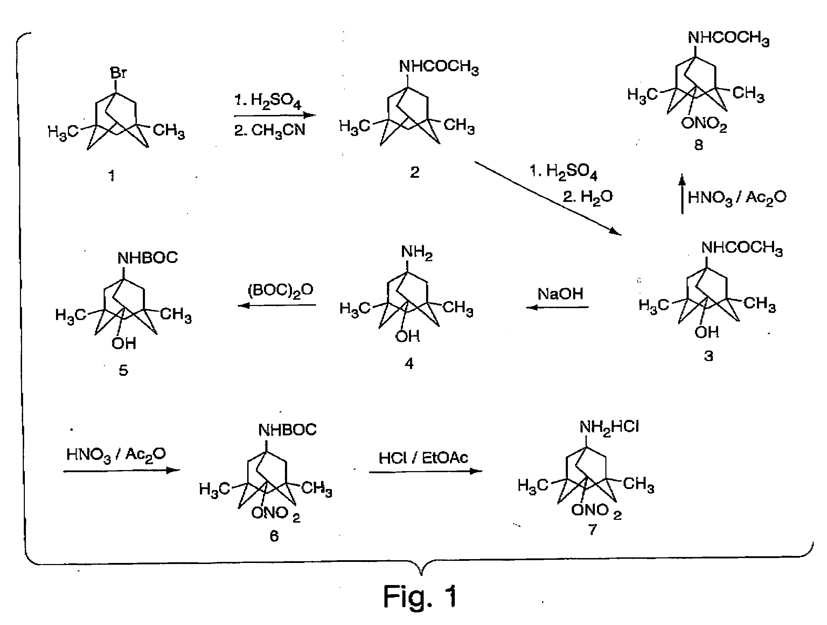

FIG. 1 shows the synthesis of an amido nitrate derivative. Compound 1, a dimethyl-bromo-adamantane, was treated with sulfuric acid and acetonitrile to afford the dimethyl amido compound 2. Amide 2 was reacted with sulfuric acid and water, providing amido alcohol 3, which was nitrated using nitric acid and acetic anhydride to form compound 8.

FIG. 1 also shows the synthesis of an amino nitrate derivative. Compound 3 was deprotected with sodium hydroxide, affording amino alcohol 4. The amine group of compound 4 was protected with (BOC)₂O to form the carbamate alcohol 5. Carbamate 5 was nitrated using nitric acid and acetic anhydride, providing nitrate 6, which was deprotected upon treatment with hydrochloric acid to form amino nitrate hydrochloride salt 7.

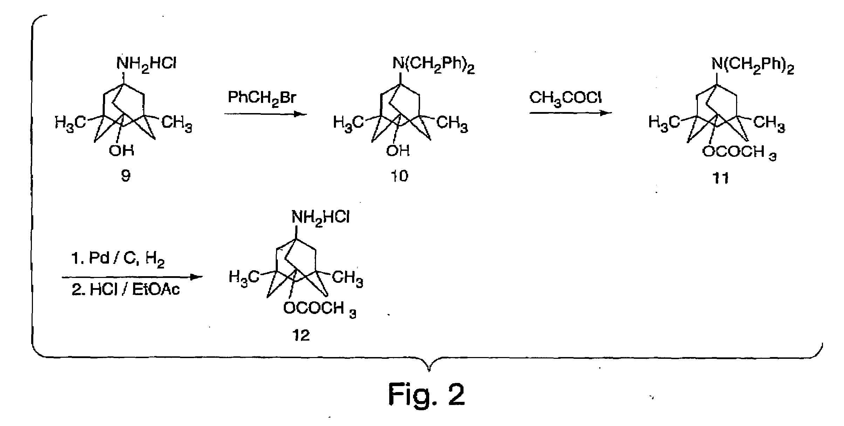

FIG. 2 shows the synthesis of an amino ester derivative. Amino alcohol 9 was alkylated with two equivalents of benzyl bromide to afford protected amino alcohol 10. Compound 10 was acetylated, yielding ester 11. Ester 11 was subjected to hydrogenation and then acidified to provide the amino alcohol hydrochloride salt 12.

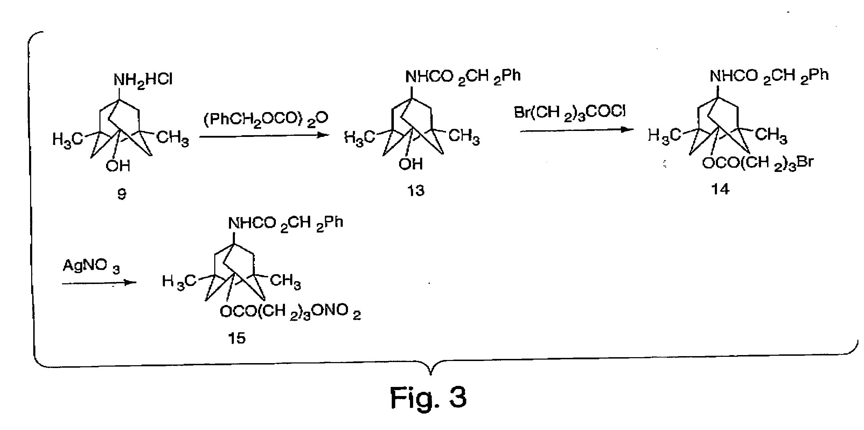

FIG. 3 shows the synthesis of a carbamate nitrate-ester derivative. Amino alcohol 9 was protected upon treatment with (PhCH₂OCO)₂O, yielding carbamate 13. Carbamate 13 was esterified using a haloalkyl acid chloride to provide compound 14, which was subjected to nucleophilic displacement with AgNO₃, affording carbamate nitrate-ester 15.

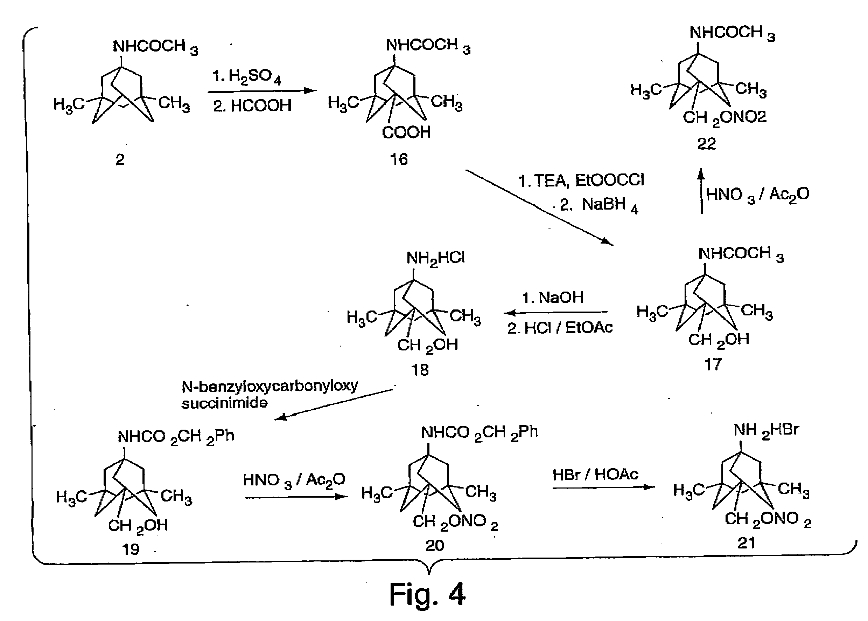

FIG. 4 shows the synthesis of an amido alkyl-nitrate derivative. Amide 2 was reacted with sulfuric acid and formic acid to form amido acid 16. Treatment of compound 16 with triethyl amine and ethyl chloroformate, providing a mixed anhydride, followed by reduction with sodium borohydride yielded amido alkanol 17. Nitration of 17 using nitric acid and acetic anhydride afforded amido alkyl-nitrate 22.

FIG. 4 also shows the synthesis of an amino alkyl-nitrate derivative. Amido alkanol 17 was deprotected with sodium hydroxide and acidified with hydrochloric acid to provide 18. The amine group of compound 18 was protected upon reaction with N-benzyloxycarbonyloxysuccinimide, forming carbamate 19, and subsequently nitrated using nitric acid and acetic anhydride to yield carbamate alkyl-nitrate 20. The carbamate of compound 20 was removed with hydrobromic acid and acetic acid, providing amino alkyl-nitrate 21.

There are a number of compounds that are preferable intermediates for the synthesis of either amido or amino alkyl-nitrate derivatives. Such compounds include amido acid 16, amido alkanol 17 and amino alcohol hydrochloride salt 18.

The compounds and compositions of the present invention can be used to manufacture medicaments to treat a number of neuropsychiatric disorders and disease states, such as disorders arising from trauma, ischemic or hypoxic conditions including stroke, hypoglycemia, cerebral ischemia, cardiac arrest, spinal cord trauma, head trauma, perinatal hypoxia, cardiac arrest and hypoglycemic neuronal damage. Neurodegenerative disorders such as epilepsy, Alzheimer's disease, Huntington's disease, Parkinsonism, and amyotrophic lateral sclerosis can also be treated. Other neuropsychiatric diseases or disorders that can be ameliorated through administration of the compounds and compositions include, without limitation, the following: depression, bipolar disorder, anxiety, convulsion, pain, schizophrenia, muscle spasms, migraine headaches, urinary incontinence, nicotine withdrawal, opiate tolerance and withdrawal, emesis, brain edema, tardive dyskinesia, AIDS-induced dementia, ocular damage, retinopathy, cognitive disorders, and neuronal injury associated with HIV-infection such as dysfunction in cognition, movement and sensation.

### Assays for Therapeutic Effects

Assessments and inventories for determining a therapeutic effect in the treatment of neuropsychiatric disorders can generally be found in Diagnostic and Statistical Manual of Mental Disorders, 4^{th} Ed., American Psychiatric Press, (1994). Patient assays and assessments in combination with more empirical tests such as serum or CSF plasma glutamate levels, MRS, MRI, and PET provide the best methods for determining the efficacy of a treatment regimen with NMDA receptor antagonist compounds. These assessments can be combined with other criteria, i.e., weight gain or loss, the ability to acquire or maintain employment, the ability to interact in social situations, as well as more subjective inventories, such as drug craving behavior, an increase or decrease of libido or energy, and generalized assessments of well-being, to give a more complete analysis of the patient's response to therapy.

### CSF (Cerebral Spinal Fluid) and Plasma Levels of Glutamate in Depressed Patients

A therapeutic effect from the compounds used to modulate NMDA receptor activity may be determined by assaying for a reduction of glutamate or glutamine levels in plasma and cerebral spinal fluid. Glutamate plasma levels are known to be higher in depressed patients compared with a population control group (see, Kim *et al,* 1982; Mathis *et al.,* 1988, Mauri *et al.,* (1998), and Berk *et al.,* (2000), Levine *et al.,* (2000), and Castillo *et al.,* (2000), incorporated herein by reference. Although memantine does not block glutamate release but only the effect of glutamate on its NMDA receptor, that is, glutamate levels may remain elevated but memantine may simply block the effects of the elevated glutamate. However, because memantine will block cell death and hence abnormal glutamate release in that manner, plasma and CSF glutamate levels often decrease in response to patient therapy.

### Patient Assessments:

Efficacy of therapy with NMDA receptor antagonists can also be determined by administering one or more of the inventories described below. An improvement in patient score generally correlates with a reduced pathophysiological state and an improvement in the neuropsychiatric disease state.

*The MADRS* (Montgomery and Asberg, 1979, incorporated herein by reference) is a 10-item instrument used for the evaluation of depressive symptoms in adults and for the assessment of any changes to those symptoms. Inter-rater reliability of the scale is high and scores correlate significantly with those of the HAMD (discussed herein). Each of the 10 items are rated on a scale of 0 to 6, with differing descriptors for each item. These individual item scores are added together to form a total score, which can range between 0 and 60 points. An improvement in patient score of greater than 5% after administration of the NMDA antagonist compound indicates a decrease in the pathophysiology of the neuropsychiatric disorder, while an improvement in patient score of 10% following a therapeutic regimen with the compound indicates the achievement of a therapeutic effect.

*HAMD* (Hamilton, 1960, *see also,* de Montigni *et al.,* 1999; Versiani *et al*., 2000; Shelton *et al*., 2001, incorporated herein by reference) is a widely used observational rating measure of depression severity. The 21-item version of this scale (HAMD) is administered to assess the severity of depression and its improvement during the course of therapy. It assesses both the presence and severity of individual signs and symptoms characterizing depression without psychotic features. The total score is the sum of 21 items, and it ranges from 0 to 65. An improvement in patient score of greater than 5% after administration of the NMDA antagonist compound indicates a decrease in the pathophysiology of the neuropsychiatric disorder, while an improvement in patient score of 10% following a therapeutic regimen with the compound indicates the achievement of a therapeutic effect

*The CORE Assessment of Psychomotor Change* (Parker and Hadzi-Pavlovic, 1996) is comprised of 18 signs (observable features), which are rated by the clinician at the end of the interview. Each sign is rated on a four-point scale (0-3). Summing subsets of the items produces scores on three dimensions found to underlie psychomotor change: non-interactiveness, retardation and agitation. A total score from the items can be used to assign patients to melancholic or non-melancholic subtypes. The CORE Assessment of Psychomotor Change is not a diagnostic measure. It is a sub-type system to be used when a diagnosis of primary depression has been made and to divide melancholic versus non-melancholic type. A change in classification from a melancholic subtype to a non-melancholic subtype indicates a decrease in the pathophysiology of the disease state.

*The Hamilton Psychiatric Rating Scale for Anxiety* (HAM-A) is a widely used observational rating measure of anxiety severity. The scale consists of 14 items. Each item is rated on a scale of 0 to 4. This scale is administered to assess the severity of anxiety and its improvement during the course of therapy. The HAM-A total score is the sum of the 14 items and the score ranges from 0 to 56. An improvement in patient score of greater than 5% after administration of the NMDA antagonist compound indicates a decrease in the pathophysiology of the neuropsychiatric disorder, while an improvement in patient score of 10% following a therapeutic regimen with the compound indicates the achievement of a therapeutic effect in treating anxiety.

*The YMRS* (Young *et al,* 1978, incorporated herein by reference) consists of 11 items. Items 5, 6, 8, and 9 are rated on a scale from 0 (symptom not present) to 8 (symptom extremely severe). The remaining items are rated on a scale from 0 (symptom not present) to 4 (symptom extremely severe). Items 5, 6, 8, and 9 (irritability, speech, content and disruptive-aggressive behavior) are given twice the weight of the remaining 7 in order to compensate for the poor condition of severely ill patients. The YMRS total score ranges from 0 to 60 and is the primary efficacy parameter. The YMRS scale is obtained should hypomanic/manic symptoms develop during therapy. An improvement in patient score of greater than 5% after administration of the NMDA antagonist compound indicates a decrease in the pathophysiology of the neuropsychiatric disorder, while an improvement in patient score of 10% following a therapeutic regimen with the compound indicates the achievement of a therapeutic effect.

The *PANSS* ratings are derived from a formal, semi-structured, 30- to 40-minute clinical interview and additional sources of information. The 30 items in the PANSS are rated on a seven-point scale (1= absent, 7 = extreme). Seven items are grouped to form a Positive Scale, which assesses features exhibited in schizophrenia that are not present in those with a normal mental state. Another seven items constitute the Negative Scale, assessing features that are absent in schizophrenia but that would be present in those with a normal state. Based on the differential between these scales, a bipolar Composite Scale specifies the degree of preponderance of one syndrome over the other. Finally, a fourth index, the General Psychopathology Scale, gauges the overall severity of the disorder by summation of the remaining 16 items. Three supplementary items assess the risk of aggression. The PANSS rating provides a method of evaluating patients should psychotic symptoms develop during the course of therapy. Psychotic symptoms have been reported to occur with the use of NMDA antagonists, and any compound that modulates the glutamate neurotransmission pathway should be evaluated for its propensity to develop psychotic symptoms.

*The CGI scale* (National Institute of Mental Health, 1976, incorporated herein by reference) is a three-item scale that assesses treatment response in psychiatric patients. The administration time is 5 minutes. This scale consists of three items: Severity of Illness (item 1); Global Improvement (item 2); and Efficacy Index (item 3), Item 1 is rated on a seven-point scale (1 = normal, 7 = among the most extremely ill patients); as in item 2 (1 = very much improved, 7 = very much worse). Each includes an additional response of not assessed. Item 3 is rated on a four-point scale (from none to outweighs therapeutic effect). Items 1 and 3 are assessed based on the previous week's experience. Item 2 is assessed from the period since the initiation of the current treatment. An improvement in patient score of greater than 5% after administration of the NMDA antagonist compound indicates a decrease in the pathophysiology of the neuropsychiatric disorder, while an improvement in patient score of 10% following a therapeutic regimen with the compound indicates the achievement of a therapeutic effect.

### Magnetic Resonance Spectroscopy

More empirical data on patient response to therapy can be obtained through magnetic resonance Spectroscopy, and PET. Neuronal injury is associated with, for example, decreased N-acetyl-aspartate (NAA) peaks on MR spectroscopy. This kind of chemical shift can be followed over the course of patient treatment to monitor the progression of the neuropsychiatric disorder as well as to formulate dosage regimens.

Quantitative single voxel [¹H]-MRS exams are performed using a 3.0T clinical scanner (GE Signa/Horizon 5.6, Milwaukee). A Stimulated Echo Acquisition Mode (STEAM) pulse sequence is used to acquire spectra using the following acquisition parameters and also includes unsuppressed water reference scans for metabolite quantitation: an echo time of 30 msec, a modulation time of 13.7 msec, a repetition time of 2 sec, 8 step phase cycle, 2048 points, a spectral width of 2500 Hz, and 128 averages for a total acquisition time of approximately 5 minutes. Spectra will be acquired from approximately 8cc regions of interest (ROIs) in the frontal, temporal, parietal, and occipital lobes, Compounds identified in the short echo [¹H]-MRS human brain studies include the neuronal marker, N-acetyl-aspartate (NAA.), glutamine/glutamate/GABA (Glx), creatine/phosphocreatine (Cr), choline compounds (Cho) and myo-Inositol (ml), The area under each of the resonances is proportional to the concentration of the specific neurochemical compound. A decrease in the pathophysiology of the neuropsychiatric disorder in response to memantine administration results in increases in, for example, the NAA peak, signifying improvement in neuronal function.

Individual peak areas are fit using time domain analysis software. The concentrations of each compound are reported in arbitrary quantitative units as a ratio to brain water concentration (x10⁴/water). This water referencing method has been used in the field for over a decade and has been validated by a number of research groups. The analysis software is public domain (http: //carbon.uab.es/mruiwww) and eliminates much of the subjectivity previously involved in determining spectral peak areas using older methods. Briefly, the software performs an automated fit of the unsuppressed water peak to determine its peak area and also uses the phase of the water peak to apply an automated zero order phase correction to the metabolite data. Following this, the user enters *a priori* information regarding the metabolite data in order to give the software starting values for its fitting process. The *a priori* information given includes the expected chemical shifts for each of the major chemical compounds appearing in the typical proton brain spectrum as well as a starting linewidth determined by the corresponding water linewidth. The chemical shift values given to the program are based on literature values, which are 2.02 ppm for NAA, 2.3 ppm for the Glx complex, 3.03 ppm for Cr, 3.22 for Cho, and 3.56 for ml. With this input, the software will then attempt to fit the metabolite spectrum and display its results both visually and in a file, which can be pasted into a spreadsheet analysis program. In order to achieve reliable fits for ml, one must also fit the additional and partially overlapping peaks of Cr and Glx in the immediate area of mI. The visual and quantitative results are inspected for goodness of fit and either accepted or reiterated again for improvement. Most spectra (approx. 80%) require only 1 iteration to achieve a satisfactory fit with the majority of the others being successful after 2 iterations. A fit is accepted if the residual shows predominately unstructured noise. The areas of the water peaks and metabolite peaks are entered into a spreadsheet. The data for the individual metabolite peaks are then multiplied by 10,000 (factor chosen for convenience in reporting data) and then divided by the unsuppressed brain water peak area. Quantitative metabolite concentrations are reported in arbitrary units as (x10⁴)/water. Water and metabolite relaxation effects are not corrected for with this technique because obtaining these values on each patient would be time prohibitive (measurement time would take an additional 2 hours in each subject). Acquisition parameters are utilized, which minimize the uncertainty in neurochemical concentration estimates due to relaxation effects. Specifically, a short echo time of 30 msec minimizes T2 signal decay and a standard repetition time of 2 sec minimizes T1 error resulting from collecting spectra under less than fully relaxed conditions. This is a common trade-off in clinical situations.

### PET and the Functional Anatomy of Depression: Implications for Glutamatergic Transmission

PET imaging studies of depressed subjects with MDD and BPD have demonstrated abnormalities of regional cerebral blood flow (CBF) and glucose metabolism, which suggest regions where glutamatergic transmission may be abnormal in depression. The glucose metabolic signal (which correlates tightly with CBF during physiological activation) predominantly reflect glutamatergic transmission (Magistretti *et al.,* 1995). In the depressive subgroups, a consistent pattern of abnormalities has emerged in the neural circuitry implicated in emotional processing by other types of experimental evidence. Specifically, the major depressive episode is associated with elevated glucose metabolism in limbic areas such as amygdala and ventral anterior, cingulate cortex, and cortical and subcortical areas which have extensive anatomical connections with these regions, such as the anterior insula, the orbital cortex, the posterior cingulate, the medial thalamus, and the ventral striatum (reviewed in Drevets, 2000).

### Specificity of Elevated Limbic-Thalamo-Cortical (LTC) Activity

Metabolism in the amygdala and anterior cingulate is abnormally elevated in MDD subgroups who were responsive to sleep deprivation. Medicated, remitted MDD subjects who relapsed during serotonin depletion have typically higher baseline amygdala and orbital cortex metabolism than those who did not relapse. Other studies of unmedicated depressives with MDD also have reported increased CBF and metabolism in the orbital cortex relative to healthy controls, and longitudinal studies in which depressives are imaged before and during treatment consistently show that CBF and metabolism decrease in the orbital cortex, the ventromedial PFC, the pregenual and subcallosal ACC and the anterior insula following effective antidepressant drug therapy, ECT, phototherapy, repeated transcranial magnetic stimulation (rTMS), and sleep deprivation (reviewed in Drevets *et al.,* 1999, Drevets, 1999, incorporated herein by reference), Treatment responsiveness to memantine can be predicted by elevated limbic-thalamo-cortical activity in the baseline, depressed-unmedicated, condition. Such an assessment allows for the identification of a sub-phenotype of MDD that is more likely to benefit from treatment with compounds that reduce glutamatergic transmission.

### Initiating Patient Scans

Subject preparation consists of intravenous catheterization. PET scans are acquired using a GE Advance (35 contiguous slices with 4.25 mm plane separation; 3D resolution 6 to 7 mm FWHM, 3D acquisition mode). The initial emission scan is acquired over the heart, so the subjects are moved feet first into the whole body scanner. First, a 2-min transmission scan using rotating rods of ⁶⁸Ge/⁶⁸Ga with electronic windowing around the rods to minimize scatter is obtained over the chest. This scan is immediately reconstructed to guide repositioning of the, scanner gantry so that it is centered over the heart. After repositioning the subject, an approximately 8 min transmission scan is acquired for attenuation correction of the cardiac emission scan during the tracer uptake period. Following this transmission scan, 4.5 mCi of [¹⁸F] fluorodeoxyglucose (FDG) is administered by *i.v*. slow bolus injection (over 2 min). A 35 min long dynamic 2D emission scan is acquired as 10 x 30 second frames followed by 10 x 3 minute frames.

Following this scan the subject will get up off the scanner bed and the bed will be fitted with the head-holder. The subject is positioned head first into the scanner and the head is immobilized using a thermoplastic mask, which constrains head position at multiple surfaces (e.g., forehead, temporal and occipital surfaces, mandible) to reduce the likelihood of movement. The cerebral emission scan is acquired as subjects rest with eyes-closed. A 2 minute transmission scan is acquired and immediately reconstructed so that the primary structures of interest are located approximately in the center of the field-of-view. A second transmission scan (8 minutes) is acquired for attenuation correction of the emission data. A 10-minute emission scan is initiated 45 min after FDG injection. Venous blood sampling at 5 minute intervals is initiated at 45 min post FDG injection. The radioactivity of the plasma and whole blood is counted. Three venous samples are also obtained to measure plasma glucose.

The post-treatment scan is acquired using identical methods. Subjects are repositioned in the scanner by aligning laser lines projected from the scanner gantry onto markings on the hardened thermoplastic mask worn during the initial scan so that the head position is approximately the same in all frames.

### Assessment of Ventral Striatum in PET Images

An important region for studies of depression that is small relative to the spatial resolution of PET is the ventral striatum, which contains the nucleus accumbens. In primates the cells with connectional and histochemical features of the accumbens blend with those of the anteroventral putamen and ventromedial caudate, such that the nucleus accumbens lacks distinct microscopic and macroscopic borders (Heimer and Alheid, 1991, incorporated herein by reference). This anteroventral portion of the striatum (AVS) is innervated by the amygdala and the orbital and medial PFC areas implicated in reward-related and emotional processing, while the dorsal caudate and dorsal putamen primarily receives afferent connections from cortical areas involved in sensorimotor function (Everitt *et al.,* 1989; Haber *et al.,* 1995; Öngür and Price, 2000; Selemon and Goldman-Rakic, 1985, incorporated herein by reference).

In previous PET studies of dopamine (DA) D2 /D3 receptor binding aimed at understanding the relationship between emotion and ventral striatal DA release, PET measures of the change in endogenous DA following dextroamphetamine (AMPH) were correlated with the associated hedonic response in humans across subregions of the striatum (Drevets et al. 1999, 2001). PET measures of [¹¹C] raclopride specific binding to DA D2/D3 receptors obtained before and after AMPH injection (0.3 mg/kg i.v.) in healthy subjects showed that the change in binding potential (delta BP) induced by AMPH was significant in the AVS [comprised of accumbens area, ventromedial caudate, and anteroventral putamen; p <0.005] but not the dorsal caudate (DCA; t = -1.45). The delta BP in the AVS was greater than that in the DCA [p< 0.05] and the middle caudate (MCA; p< 0.01), and similar to that in the ventral putamen (VPU). The change in euphoria ratings correlated with delta BP in the AVS (r = -0.95, p= 0.001) but not in the DCA (r = +0.30, n.s.). The difference between these correlation coefficients was significant (p< 0.01). Changes in euphoria correlated with delta BP in the VPU [r = -0.77; p< 0.05] but not in the DPU (r =+0.25), the MCA (r = -0.61), or the whole striatum (r = -0.50).

Because of PET's limited spatial resolution, regional measures are affected by radioactivity spilling in from surrounding tissues and dilutional effects from adjacent structures (Links *et al.,* 1996). Measured signals from the DCA and the AVS are easily differentiated, but the AVS and ventral putamen results are weakly correlated (Drevets *et al.,* 1999). This reflects the greater axial separation between the AVS and the DCA (7 to 12 mm in humans) relative to the axial scanner resolution, as compared with the smaller anterior-posterior separation of the AVS and putamen relative to the transverse resolution (e.g., the mean AVS volumes were 2.77 + 0.722 mL in humans; Drevets et al. 2001). The volumetric resolution of a 1.25 mm point radioactivity source in a Siemens HR+ (similar to that of the GE Advance) has a measured FWHM resolution of 5.3 mm axial and 6.6 mm transverse, yielding a volumetric resolution of 0.23 mL. This volume is only 8.3 % of the mean AVS volume (2.77 ±0.722 mL) measured in MRI images from healthy humans. The axial resolution of 5.3 mm FWHM implies that pixels located greater than 11 mm from the edge of the AVS will have virtually no effect on PET measures from the AVS. The center-to-center separations between the AVS and the DCA and the DPU are well over this distance. In humans, measured signals from the AVS can therefore be easily differentiated from those of the DCA and the DPU, and will be weakly influenced by those from the VPU and MCA.

### Metabolic Activity in the Anteroventral Striatum in Depression

Demonstrating differential regional metabolic abnormalities across subregions in the current study thus depends upon showing that the mean difference between two groups is greater in the AVS than in the DCA, and that this difference is not accounted for by an even greater difference in the MCA or the VPU (Drevets et al.1999,2001). The ability to assess relative differences in radiotracer concentration across conditions in ROI separated by less than the FWHM resolution is central to PET's utility in localizing voxels of maximal difference in brain mapping studies (Fox *et al*., 1986; Friston *et al*., 1996, incorporated herein by reference).

MRI-based ROI analysis can be used to assess glucose metabolism in these striatal subregions between unipolar depressives and healthy controls. In the controls the coefficient of variance (SD/ mean) for normalized glucose metabolism in the AVS ranges between 6-7% as measured using PET cameras with similar sensitivity and resolution to that planned for the current study. Regional glucose metabolism is increased over this 6-7% range in the depressives compared to controls in the AVS (p<0.05) but not in the MCA, DPU. DCA, or VPU, A change in regional glucose metabolism indicates a therapeutic effect, i.e., any decrease beyond the 6-7% variance in regional glucose metabolism in a patient indicates a therapeutic effect.

### Example 1. Synthesis of 1-acetamido-3, 5-dimethyl-7- hydroxyadamantane (3)

Fuming H₂SO₄ (3 mL) was added to 1-acetamido-3, 5-dimethyladamantane (0.2 g) at 0 °C under nitrogen and the reaction mixture was stirred at 0 °C for 1h. The reaction mixture was poured onto ice (10 g) and the product was extracted with ether (10 mL x 4), The combined ether solution was washed with brine (10 mL) and water (10 mL). The solution was dried using sodium sulfate. The solvent was removed *in vacua* and, after crystallization on standing, 70 mg of white product was obtained. Pure product was obtained by recrystallization in ether. ¹H NMR (DMSO-d₆, ppm): 7.30 (brs, 1 H, NH), 4.37 (brs, 1 H, OH), 1.72 (s, 3 H, COCH₃), 1.65 (s, 2 H), 1.47 (s, 4 H), 1.24-1.14 (dd, 4 H, J = 11.2, 23.9 Hz), 0.99 (s, 2 H), 0.82 (s, 6 H, 2 x CH3). m. p. 194-195 °C. Anal, (C₁₄H₂₃NO₂), C.H.N.

### Example 2. Synthesis of I-amino-3, 5-dimethyl-7-hydroxyadamantane hydrochloride (4)

1-Acetamid-3, 5-dimethyl-7-hydroxyadamantane (0.4 g) and NaOH (1.1 g) were added to diethylene glycol (7 ml) and the reaction mixture was heated to 175°C for 15 h. After cooling to room temperature, ice (10 g) was added and the product was extracted with ether (10 mL x 4). The combined ether solution was washed with brine (10 mL) and water (10 mL). The solution was dried using sodium sulfate. The solvent was removed *in vacuo* and, after crystallization on standing, 250 mg of white product was obtained. HCl in ethyl acetate was added to convert the free base to HCl salt, ¹HNMR (DMSO-d₆, ppm); 8.12 (brs, 2H,NH), 4.72(brs,1H,OH), 1.58(s,2H), 1.40-1.31(dd,4H,J=12,3,21.6Hz), 1.23 (s, 4 H). 1.08-0.98 (dd, 2 H, J =12.6, 23.3 Hz), 0,88 (s, 6 H, 2 x CH3). m. p. 28 1-282 °C. Anal. (C₁₂H₂₂NOCl+0.5 H₂O), C.H.N.

### Example 3. Synthesis of 1-tert-butylcarbamate-3,5-dimethyl-7-hydroxyadamantane (5)

1-Amino-3, 5-dimethyl-7-hydroxyadamantane (100 mg) was dissolved in tetrahydrofuran (2 mL), Triethylamine (180 ml), di-*tert*-butyl dicarbonate (336 mg) and dimethylaminopyridine (2 mg) were added sequentially. The reaction mixture was stirred at room temperature for 3 h and then 0.5 N NaOH (2 mL) was added, The reaction mixture was stirred overnight. Triethylamine was removed *in vacua* and ether was added. The ether solution was washed with 0.1 N HCl and brine. The solution was dried using sodium sulfate. Solvent was removed *in vacuo* and 60 mg of product was obtained after crystallization on standing in other. ¹HNMR (DMSO-d₆, ppm): 6.35 (brs, 1H, NH), 4.35 (brs, 1H, OH), 1.59 (s, 2 H), 1.40 (s, 4 H), 1.35 (s, 9 H, 3 x CH3), 1.22-1.13 (dd, 4H, J = 11.1, 20.6 Hz), 0.99 (s, 2H), 0.82 (s, 6 H, 2 x CH3).

### Example 4. Synthesis of 1-tert-butylcarbamate-3,5-dimethyl-7-nitrateadamantane (6)

A cooled (0°C) acetyl nitrate (0.08 mL, from a mixture of fuming HNO₃ and acetic anhydride (1: 1.5/v: v) was added to a dichloromethane (1 mL) solution of 1*-tert-*butylcarbamate-3, 5-dimethyl-7-hydroxyadamantane (40 mg) at 0 °C under nitrogen and the reaction mixture was stirred at 0 °C for 15 minutes. 1 N sodium hydrogen carbonate solution (5 mL) was added and the product was extracted with dichloromethane (10 mL). The dichloromethane solution was washed with water (10 mL x 3). The solution was dried using sodium sulfate. The solvent was removed *in vacua* to afford an oily product (30 mg). ¹HNMR (DMSO-d₆, ppm): 6.66 (brs, 1 H, NH), 2.14 (s, 2 H), 1.70 (s, 2 H), 1.69 (s, 2 H), 1.63-1.60 (d, 2H, J= 12.3 Hz), 1.46-1.43 (d,2H, 3 = 12.2 Hz), 1.36 (s*,* 9 H, 3 x CH3), 1.17-1.08 (dd, 2 H, 1 11.4, 22.6 Hz), 0.91 (s, 6H, 2 x CH3). High resolution MS calculated for C₁₇H₂₈N₂O₅Na(MS + Na): 363.1895. Found 363.1908.

### Example 5. Synthesis of I-aniino-3,5-dimethyl-7-nitrateadamantane hydrochloride (7)

3 N HC1 in ethyl acetate (0.5 mL) was added to 1-*tert*-butylcarbamate-3, 5-dimethyl-7-nitrateadamantane (40 mg). The reaction mixture was stirred at room temperature for 30 minutes. The precipitate was filtered and the product was washed with ether. A pure white product was obtained (35 mg). ¹H NMR (DMSO-d₆, ppm): 8.36 (brs, 2 H, NH), 2.15 (s, 2 H), 1.69 (s, 4 H), 1.57-1.44 (dd, 4 H, J = 12.2, 32.8 Hz), 1.26-1.10 (dd, 2 H, 3 = 12.0, 44.3 Hz), 0.96 (s, 6 H, 2 x CH₃). m. p. 225-226 °C. MS (MS + H⁺): 241. Anal. (C₁₂H₂₁N₂O₃Cl), C.H.N.

### Example 6. Synthesis of 1-acetamido-3, 5-dimethyl-7-nitrateadamantane (8)

To acetic anhydride (0.3 mL) at 0 °C under nitrogen was added fuming HNO₃ (0.2 mL). After stirring for 5 minutes at 0 °C, 1-acetanudo- 3,5-dimethyl-7-hydroxyadamantane (50 mg) was added and the reaction mixture was stirred at 0 °C for 1 h. The reaction mixture was poured into cold (0 °C) 1 N sodium hydrogen carbonate solution (20 mL) and the product was extracted with ether (10 mL). The ether solution was washed with water (10 mL x 3). The solution was dried using sodium sulfate. The solvent was removed *in vacua* and 31 mg of product was obtained. ¹H NMR (DMSO-d₆, ppm): 7.52 (brs, 1 H, NB), 2.23 (s, 2 H), 1.73-1.66 (m, 9 H, COCH₃, 3 x CH₂), 1.51-1.47 (m, 2 H), 1.15-1.13 (m, 2 H), 0.92 (s, 6 H, 2 x CH₃). m. p. 152-153 °C. Anal. (C₁₄H₂₂N₂O₄), C.H.N.

### Example 7. Synthesis of 1, 1-dibenxylamino-3, 5-dimethyl-7-hydroxyadamantane (10)

To a solution of 1-amino-3, 5-dimethyl-7-hydroxyadamantane hydrochloride (100 mg) in DMF (2 mL) was added benzyl bromide (0.16 mL) and sodium carbonate (200 mg). The reaction mixture was stirred overnight. The product was extracted with dichloromethane (10 mL) and washed with water (20 mL x 2). The organic phase was dried using sodium sulfate and the solvent was removed *in vacua.* The product was purified by flash column chromatography eluting with ethyl acetate and hexane (1/2, v/v) to afford 124 mg of white solid (76% yield). ¹H NMR (DMSO- d₆, ppm): 7.3 1-7.04 (m, 10 H, 2 x C₆H₅), 4.32 (1 H, OH), 3.71 (s, 4H, 2 x C₆H₅,CH₂), 1.44(8, 2 H), 1.35-1.27 (m, 4 H), 1.22-1.13 (dd, 4H, J= 11.8, 21.2 Hz), 0.97 (s, 2H), 0.81 (s, 6 H, 2 x CH₃).

### Example 8. Synthesis of 1-amino-3,5-dimethyl-7-acetoxyadamantane hydrochloride (12)

To a solution of 1,1-dibenzylamino-3, 5-dimethyl-7-hydroxyadamantane (50 mg) in DMF (0.4 mL) was added dichloromethane (2 mL). Acetylchloride (1 mL) was added at 0 °C under nitrogen and the reaction mixture was stirred 5 overnight. Saturated sodium carbonate solution (5 mL) was added. The product was extracted with dichloromethane (10 mL) and washed with water (20 mL x 2). The organic phase was dried using sodium sulfate and the solvent was removed *in vacua.* Without further purification, the product was dissolved in methanol (10 mL). Pd/C (10%,10 mg) was added and the reaction mixture was hydrogenated at a pressure of 40 LB/inch² overnight. The mixture was filtered and solvent was removed. HCl in ethyl acetate was added and the precipitate was filtered and the solid was washed with hexane to afford 15 mg of product after drying in air. ¹H NIvIR (DMSO-d₆, ppm): 8.30 (brs, 2 H, NH₂), 2.09 (s, 2 H), 1.93 (s, 3 H, COCH₃), 1.72-1.63 (dd, 4 H, J = 12.6, 21.4 Hz), 1.50-1.39 (dd, 4 H, J =11.7, 29.6 Hz), 1.18-1.05 (dd, 2 H, J =14.1, 36.5 Hz), 0.93 (s, 6 H, 2 x CH₃).

### Example 9. Synthesis of 1-(beuzyloxycarbonyl)amino-3,5-dimethyl-7-hydroxyadamantane (13)

To a solution of 1-amino-3, 5-dimethyl-7-hydroxyadamantane hydrochloride (570 mg) in DMF (5 ml) and water (0.3 mL) was added dibenzyl dicarbonate (1.41 g) and sodium carbonate (1.3 g). The reaction mixture was stirred overnight. The product was extracted with *t*-butyl methyl ether (500 mL) and washed with water (400 mL x 2). The organic phase was dried using sodium sulfate and the solvent was removed *in vacuo.* The product was purified by flash column chromatography eluting with ethyl acetate and hexane (1/3, v/v) to afford 701 mg of white solid. 1H NMR (DMSO-d₆, ppm): 7.35-7.28 (m, 5 H, C₆H₅), 6.96 (brs,1 1 H, NH), 4.94 (s, 2 H, OCH₂), 4.41 (1 H, OH), 1.62 (s, 2 H), 1.43 (s, 4 H), 1.24-1.14 (dd, 4 H, J = 11.5, 22.0 Hz), 0.97 (s, 2H), 0.83 (a, 6 H, 2 x CH₃).

### Example 10. Synthesis of 1-(benzyloxycarbonyl)amino-3, 5-dimethyl.-7-(3-bromopropylcarbonyloxy)adamantane (14)

To a solution of 1-(benzyloxy-carbonyl)amino-3, 5-dimethyl-7-hydroxyadaniantane (100 mg) in DMF (0.4 mL) was added 4-bromobutyryl chloride (0.3 mL). The reaction mixture was stirred for 2 h at room temperature, The mixture was purified by thin layer chromatography eluting with ethyl acetate and hexane (1/2, v/v) to afford an oily product. 1H NMR (DMSO- d₆, ppm): 7.38-7.29 (m, 5 H, C₆H₅), 7.12 (bis, 1 H, NH), 4.95 (s, 2 H, OCH₂), 3.53-3.49 (t, 2 H, 3 = 6.6 Hz. COCH₂), 2.36-2.32 (t, 2 H, J = 7.7 Hz, CH₂Br), 2.10 (s, 2 H), 2.00-1.96 (m, 2 H, CH₂CH₂CH₂), 1.66 (s, 4 H), 1.59-1.41 (dd, 4 H, J =11.5, 51.7 Hz), 1.08-1.07 (d, 2 H. J = 3.8 Hz), 0.87 (s, 6 H, 2 x CH₃),

### Example 11. Synthesis of 1-(benzyloxycarbonyl)amino-3,5-dimethyl-7-(3-nitratepropylcarbonyloxy)adamantane (15)

To a solution of 1-(benzyloxy carbonyl)amino-3, 5-dimethyl-7-(3-bromopropylcarbonyloxy)adamantane in acetonitrile was added a solution of silver nitrate in acetonitrile and the reaction mixture was stirred overnight in dark. The product was extracted with t-butyl methyl ether and the solution was washed with water. The organic phase was dried using sodium sulfate arid solvent was removed to afford the nitrate compound.

### Example 12. 1-Acetamido-3,5-dimethyl-7-carboxylic acid adamantane (16)

To fuming H₂SO₄ (15 mL) in a flask cooled to 0 °C 1-acetamido-3, S-dimethyladamantane (1.0 g) was added slowly over a period of 1 h. The reaction mixture was stirred for 2 h at 0 °C. Formic acid (3 mL) was then added dropwise over 1 h. The solution was stirred at 0 °C for another 2 h. The reaction mixture was poured onto ice (100 g) slowly with vigorous stirring. The precipitate formed was filtered and washed with water to give a pure white solid (0.37 g). m.p. 26 1-262 °C.

### Example 13. 1-Acetamido-3,5-dimethyl-7-hydroxymethyladamantane (17)

Triethylamine (0.80 mL) and ethyl chloroformate (0.80 mL) were added sequentially into a suspension of 1-acetamido-3, 5-dimethyl-7-carboxylic acid- adamantane (2.0 g) in THF at 0 °C. The reaction mixture was stirred for 4 h at room temperature. The white precipitate formed was then filtered and washed with THF. NaBH₄ (2.40 g) was added to the filtrate. Water (2 mL) was added dropwise to the solution over a period of 1 h followed by addition of more water (50 mL). The organic solvent was removed under reduced pressure and the remaining aqueous solution was extracted with ethyl acetate (100 mL x 3). The combined organic extracts were washed with 0,5 N HCI twice, water, and brine. Solvent was removed *in vacua* and the product was crystallized using a solution of ethyl acetate and hexane (1/4, v/v) to give a white solid (700 mg). ¹H NMR (DMSO-d₆, ppm): 7.28 (s, 1H, NH), 4.33 (t, 1H, OH, J 5,7 Hz), 3.02 (d, 2 H, CH₂OH, 5.7 Hz),1.71 (s, 3 H, COCH₃), 1.49 (s, 6 H), 1.07-0.97 (m, 6 H), 0.96 (s, 6 H) m. p. 152-153 °C. Anal. (C₁₅H₂₅NO₂), C.H.N.

### Example 14. 1-Amino-3,5-dimethyl-7-hydroxymethyladamantane

### hydrochloride (18).

1-Acetamido-3, 5-dimethyl-7-hydroxymethyladamantane (200 mg) and NaOH (540 mg) were added to diethylene glycol (4 mL) and the reaction mixture was heated to 175 °C under nitrogen for 15 h. After cooling to room temperature, ice (5 g) was added and the product was extracted with ethyl acetate (10 mL x 6). The combined extract was washed with water (10 mL) and brine (10 mL), and dried using sodium sulfate. Solvent was removed *in vacua*. HCl in ethyl acetate was added to convert the free base to HCl salt and 102 mg of product was obtained. ¹NMR (DMSO-d₆, ppm): 8.19 (brs, 2 H), 4.54-4.51 (t, 1 H, OH, J = 5.0 Hz), 3.07-3.05 (d, 2 H, OCH₂, J = 4.6 Hz), 1.42-1.40 (m, 6 H), 1.01-0.99 (m, 6 H), 0.86 (s, 6 H). Anal. (C₁₃H₂₄NOCl+O.4 HCl), C.H.N.

### Example 15. 1-(benzyloxycarbonyl)amino-3,5-dimethyl-7-hydroxymethyl adamantane (19).

To a solution of 1-amino-3,5-dimethyl-7-hydroxymethyl adamantane (60 mg) in THF (3 mL) was added N-(benzyloxycarbonyloxy)-succinimide (74 mg) and the mixture was stirred at room temperature overnight. THF was removed and the residue was dissolved in ethyl acetate. The solution was washed with water and brine. The product was purified by thin layer chromatography eluting with ethyl acetate and hexane (1: 4, v/v) to give a white solid (80 mg). ¹H NMR (DMSO-d6, ppm): 7,33 (m, 5 H, C6H5), 6.89 (brs, 1 H, NH), 4.94 (s, 2 H, OCH2), 4.32 (t, 1H, OH, J = 5.7 Hz), 3.04 (d, 2 H, CH₂OH, J = 5.7 Hz), 1.46 (dd, 6 H), 1.04 (dd, 6 H), 0.84 (s, 6 H, 2 x CH₃).

### Example 16. 1-(benzyloxycarbonyl)amino-3,5-dimethyl-7-nitratemethyladamantane (20).

To a solution of 1-(benzyloxycarbonyl)amino-3,5-dimethyl-7-hydroxymethyladamantane (60 mg) in dichloromethane (3 mL) was added a cooled (0°C) 30 acetyl nitrate (1 mL, from a mixture of fuming HNO₃ and Ac₂O (2; 3/v: v). The reaction mixture was stirred at 0 °C for 15 minutes. A sodium bicarbonate solution (1 N, 5 mL) was added and the product was extracted with dichloromemane. The extract was washed with water (10 mL x 3). Solvent was removed *in vacua* and the residue was purified by thin layer chromatography eluting with ethyl acetate and hexane (1: 2, v/v) to give an oily product (40 mg). ¹H NMR (DMSO-d₆, ppm): 7.33 (m, 5 H, C₆H₅), 7.02 (brs, 1H, NH), 4.95 (s, 2 H, OCH2), 4.24 (s, 2 H, OCH2), 1.60 (s, 2 H), 1.55 (d, 2 H),1.44 (d, 2 H),1.12 (m, 6 H), 0.83 (s,6H,2xCH₃).

### Example 17. 1-Amino-3, 5-dimethyl-7-nitratemethyladamantane hydrobromide (21)

1-(benzyloxycarbonyl)amino-3,5-dimethyl-7-nitratemethyl-adamantane (17 mg) was dissolved in HBr/acetic acid (1 mL) and the solution was stirred at room temperature for 2 h. The reaction mixture was concentrated *in vacuo* to give a white solid which was washed with ether to afford the target product (10 mg). ¹H NMR (DMSO-d₆, ppm): 7.82 (brs, 3H), 4,30 (s, 2 H, OCH₂), 1,50 (s, 2 H), 1,39 (s, 4 H), 1.19 (s, 4H), 1.12 (a, 2 H), 0.88 (s, 6 H, 2 x CH₃).

### Example 18. Synthesis of 1-acetamido-3, 5-dimethyl-7-nitratemethyladamantane (22)

To acetic anhydride (0.3 mL) at 0°C under nitrogen was added turning 1 {N03 (0.2 mL). After stirring for 5 minutes at 0°C, 1-acetamido- 3,5-diraethyl-7-hydroxymethyladamantane (50 mg) was added and the reaction mixture was stirred at 0°C for 1 h. The reaction mixture was poured into cold (0°C) 1 N sodium hydrogen carbonate solution (20 mL) and the product was extracted with ether (10 mL). The ether solution was washed with water (10 mL x 3). The solution was dried using sodium sulfate. The solvent was removed *in vacua* and the product was crystallized in ether to afford the target product. ¹H NMR (DMSO-d₆, ppm): 7,38 (brs, 1 H, NH), 4.23 (s, 2 H, OCH₂), 1.72 (a, 3 H, COCH₃), 1.64 (s, 2 H), 1.59-1.56 (dd, 4 H), 1.20-1.06 (m, 6 H), 0.92 (s, 6 H, 2 x CH3). m.p. 154-155 0°C. Anal. (C₁₅H₂₄N₂O₄), C.H.N.

### Example 19. In vitro protection of neurons by compound 7.

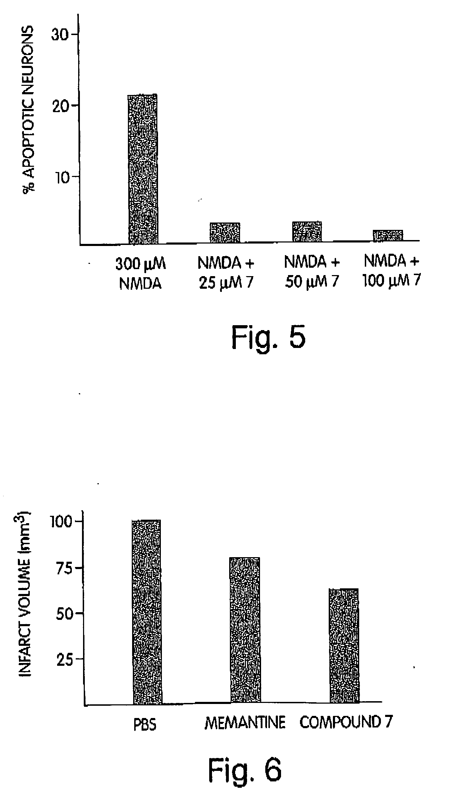

An *in vitro* model of mild NMDA-induced damage leading to apoptosis of cerebrocortical neurons was used to 30 demonstrate the protection of neurons by compound 7, Under these conditions (300 µM NMDA exposure for 20 mm, followed by washout), neuronal apoptosis was monitored 24 hours later by propidium iodide uptake and morphology of fixed, permeabilized neurons, among other techniques (Bonfoco *et al., Proc Natl Acad Sci USA (1995)* 92: 7162). NMDA induced about 20% apoptosis of neurons, and that 25-100 µM compound 7 afforded protection from this damage (P <0.001, Fig. 5). **Example 20.** *In vivo* protection by compound 7 in a murine cerebral ischemia model.

The intraluminal suture technique was used to produce a 2 hr occlusion of the middle cerebral artery (MCA), following the same protocol for focal cerebral ischomial reperfusion as published previously (Chen, *et al*., *Neuroscience* (1998) 86: 1121). However, here C57B1/6 mice were used instead of rats. For memantine the loading dose was 20 mg/kg i.p. with a maintenance dose of 1 mg/kg/12 hours, as this had been previously shown to produce parenchymal levels of 1-10 µM memantine in the brain, which was shown to be neuroprotective (Chen, *et al*., *Neuroscience* (1998) 86: 1121). To produce a neuroprotective concentration of compound 7, the loading dose was 100 mg/kg i.p. and the maintenance dose was 40 mg/kg i.p. every 12 hr. In each case, drug or vehicle control was initially administered 2 hr after MCA occlusion. Compound 7 was more neuroprotective than memantine under this paradigm (Fig. 6). The animals were sacrificed and analyzed with TTC staining 48 hr after MCA occlusion (Chen, *et al.*, *Neuroscience* (1998) 86: 1121).

### Example 21. Vasodilation by compound 8 in a rabbit model.

New Zealand white female rabbits weighing 3-4 kilograms were anesthetized with sodium pentobarbital, 13 milligram per kilogram. Descending thoracic aorta were isolated, the vessels were cleaned of adherent tissue and the endothelium was removed by a gentle rubbing with a cotton-tipped applicator inserted into the lumen. The vessels were cut into 5 millimeter rings and mounted on stirrups connected to transducers by which changes in isometric tension were recorded (model TO3C, Grass Instruments, Quincy, Mass). Vessel rings were suspended in 20 mL of oxygenated Krebs buffer at 37°C and sustained contractions were induced with 1 µM norepinephrine. The vessels were then relaxed in a dose-dependent fashion (109 through 10⁻⁵ M compound 8). In some experiments vessels Were pretreated with methylene blue or 30 hemoglobin to block relaxations.

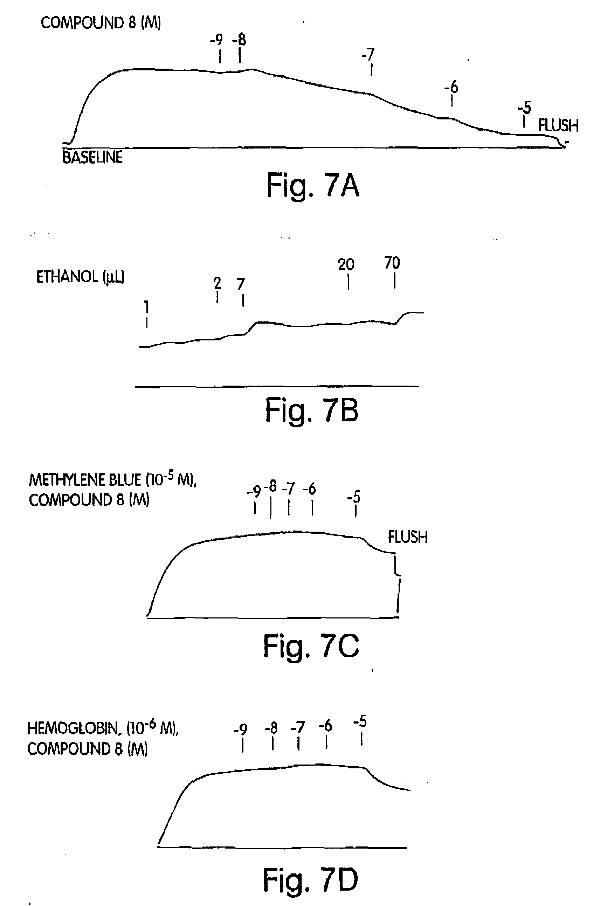

FIG. 7 shows relaxation of the precontracted aortic vessel in a dose-dependent fashion using compound 8, Relaxations were seen at 10⁻⁸ M and complete relaxation was achieved at 106 M (a). Relaxations were attenuated by methylene blue (c) and hemoglobin (d) indicating an NO-related effect. (b) is a control with solvent

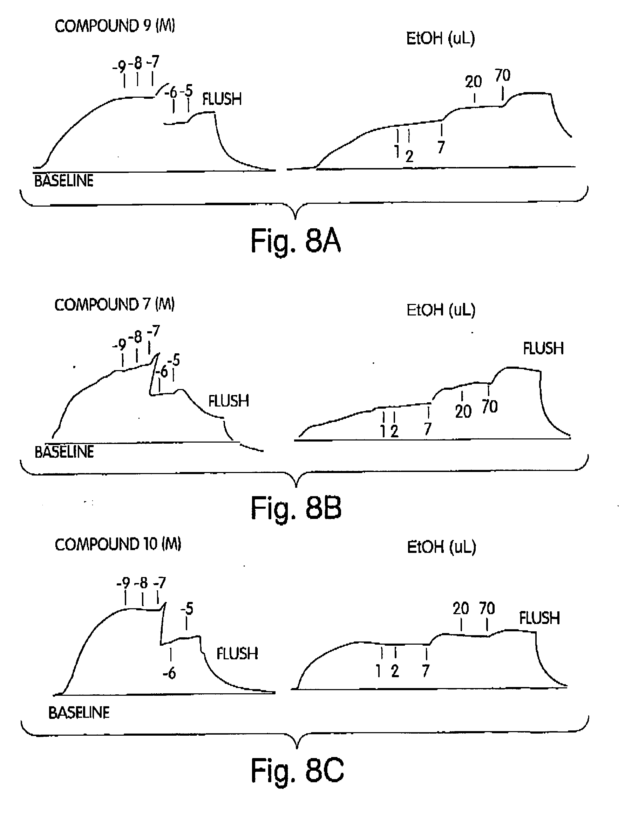

FIG. 8 shows site and specificity to derivatization of memantine. That is, compound 9 (a) and 10 (c) produced either no effect or slight contractions of blood vessels that were attributed to solvent (shown on right side). Compound 7 (b) produced modest relaxation at a 10 µM concentration.

These results demonstrate that compound 7 has vasodilator activity, in addition to NMDA-inbibitory and antiapoptic properties. Compound 7 thus acts through a unique mechanism of action that likely contributes to protective effects in models of stroke.

The scientific publications, patents or patent applications cited in the various sections of this document are incorporated herein by reference for all purposes.

### EQUIVALENTS

From the foregoing detailed description of the specific embodiments of the invention, it should be apparent that a unique method of treating neuropsychiatric disorders has been described. Although particular embodiments have been disclosed herein in detail, this has been done by way of example for purposes of illustration only, and is not intended to be limiting with respect to the scope of the appended claims which follow. In particular, it is contemplated by the inventor that various substitutions, alterations, and modifications may be made to the invention without departing from the spirit and scope of the invention as defined by the claims. For instance, the choice of the particular NMDA receptor antagonist, or the particular assay or assessment to gauge the severity or persistence of the neuropsychiatric disorder is believed to be a matter of routine for a person of ordinary skill in the art with knowledge of the embodiments described herein.

### REFERENCES

Altamura CA, Mauri MC, Ferrara A, et al. Plasma and platelet excitatory amino acids in psychiatric disorders. Am J Psychiatry 1993;150: 1731-1733.
Ambrozi L and Danielczyk W. Treatment of impaired cerebral function in psychogeriatric patients with memantine-results of a phase II double-blind study. Pharmacopsychiat 1988; 21: 144-146.
Auer DP, Putz B, Kraft E, et al. Reduced glutamate in the anterior cingulate cortex in depression: an in vivo proton magnetic resonance spectroscopy study, Biol Psychiatry. 2000 Feb 15;47(4): 305-13.
Baumann B, Danos P, Krell D, et al. Reduced volume of limbic system-affiliated basal ganglia in mood disorders: preliminary data from a post mortem study. J Neuropsych. Clin. Neurosci. 1999;11;71-78.
Bayerl JR and Gruia D. Klinsche Vergleichsstudie der Antispastika Memantin und Baclofen. {Comparative Clinical Study of the Antispastic Compounds Memantine and Baclofen}. Therapiewozhe 1985; 35: 5440-5444.
Berk M, Plein H, Belsham B, The specificity of platelet glutamate receptor supersensitivity in psychotic disorders. Life Sci 2000; 66: 2427-2432.
Berman RM, Cappiello A, Anand A, et al. Antidepressant effects of ketamine in depressed patients. Biol Psychiatry. 2000; 47: 351-4.
Bertolino A, Knable MB, Saunders RC, at al. The relationship between dorsolateral prefrontal N-acetylaspartate measures and striatal dopamine activity in schizophrenia. Biol Psychiatry 1999;45(6): 660-7.
Bhat GK, Mahesh VB, Chu ZW, et al. Localization of the N-methyl-D-aspartate R1 receptor subunit in specific anterior pituitary hormone cell types of the female rat. Neuroendocrinology 1995;62: 178-186,
Bormann J. Memantine is a potent blocker of N-methyl-D-aspartate (NMDA) receptor channel. Eur JPharmacol 1989;66: 591-592
Boyer PA, Skolnick P, Fossom LH. Chronic administration of imipramine and citalopram alters the expression of NMDA receptor subunit mRNAs in mouse brain. A quantitative in situ hybridization study. J Mol Neurosct 1998; 10: 219-233.
Bremner JD, Innis RB, Salomon RM, et al. Positron Emission Tomography Measurement of Cerebral Metabolic Correlates of Tryptophan Depletion-Induced Depressive Relapse. Arch. Gen. Psychiat. 1997;54:346-74.
Bremner JD, Narayan M, Anderson ER, et al. Hippocampal volume reduction in major depression. Am J Psychiatry 2000, 157; 115-8.
Brown ES, Rush AJ, McEwen BS. Hippocampal remodeling and damage by corticosteroids: implications for mood disorders. Neuropsychopharmacology 1999;21: 474-84
Calabrese JR, Bowden CL, Sachs GS, et al. A double-blind placebo-controlled study of lamotrigine monotherapy in outpatients with bipolar I depression. Lamictal 602 Study Group, J Clin Psychiatry 1999;60: 79-88.
Carlsson M and Carlsson A. The NMDA antagonist MK-801 causes marked locomotor stimulation in monoamine-depleted mice. J Neural Transm 1989;75: 221-226.
Castillo M, Kwock L, Courvoisie H, et al. Proton MR spectroscopy in children with bipolar affective disorder: preliminary observations. AJNR Am J Neuroradiol. 2000;21: 832-8.
Charney DS, Berman RM, Miller HL. Treatment of Depression. In The American Psychiatric Press textbook of psychopharmacology, eds. Alan F. Schatzberg and Charles B. Nemeroff, 2nd Edition, American Psychiatric Press, Inc. 1998, pp. 705-732.
Chen DF, Schneider GE, Martinou JC, Tonegawa. Bcl-2 promotes regeneration of severed axons in mammalian CNS. Nature 1997;385: 434-9.
Chen H-SV, Lipton SA. Mechanism of memantine block of NMDA-activated channels in rat retinal ganglion cells: uncompetitive antagonism. J Physiol (London) 1997;499: 27-46.
Chen H-SV, Pallegrini JW, Aggarwal SK, Lei SZ, Warach S, Jensen FE, Lipton SA. Open-channel block of NMDA responses by memantine: therapeutic advantage against NMDA receptor-mediated neurotoxicity. J Neurosci 1992;12: 4427-4436.
Chen H-SV, Wang YF, Rayudu PY, Edgecomb P, Neill JC, Segal MM, Lipton SA, Jensen FE. Neuroprotective concentrations of the NMDA open-channel blocker memantine are effective without cytoplasmic, vacuolization following post-ischemic administration and do not block maze learning or LTP. Neuroscience 1998;86: 1121-1132 NDLIG>
Choi D. Glutamate neurotoxicity and diseases of the nervous system, Neuron 1988;1: 623-634.
Cousins JP and Harper G. Neurobiochemical changes from Taxol/Neupogen chemotherapy for metastatic breast carcinoma corresponds with suicidal depression. Cancer Lett. 1996;110:163-7.
Danysz W, Parsons CG, Kornhuber J, et al. Aminoadamantanes as NMDA receptor antagonists and antiparkinsonian compounds B preclinical studies. Neurosci Biobehav Rev 1997;21: 455-468.
Ditzler K. Efficacy and tolerability of memantine in patients with dementia syndrome. A double-blind, placebo controlled trial. Arzneim Forsch/Drug Res 1991;41: 773-780
Drevets WC. Prefrontal cortical-amygdala metabolism, in major depression. Ann N Y Acad Sci 1999;29;877: 614-37
Drevets WC. Neuroimaging Studies of Mood Disorders: Implications for a neural model of major depression. Biol Psychiatry 2000;48: 813-829, 2000
Drevets WC, Gautier CH, Price JC, et al. Amphetamina-induced dopamine release in human ventral striatum correlates with euphoria Biol Psychiatry 2001;49: 81-96
Drevets WC, Price JL, Simpson JR, et al. Subgenual prefrontal cortex abnormalities in mood disorders, Nature 1997;386(6627): 824-7
Drevets WC, Price JL, Videen TO, et al. Metabolic abnormalities in the subgenual prefrontal cortex and ventral striatum in mood disorders. Soc Neurosci Abstr 1995;21(1); 260.
Duman RS, Malberg J, Nakagawa S, et al. Neuronal plasticity and survival in mood disorders, Biol Psychiatry 2000,48:732-9
Ebert D, Feistel H, Barocka A et al. Effects of sleep deprivation on the limbic system and the frontal lobes in affective disorders: A study with Tc-99m-HMPAO SPBCT. Psychiatry Res: Neuroimaging 1991;40: 247-251
Eisenberg E, LaCross S, Strassman AM. The effects of the clinically tested NMDA receptor antagonist memantine on carrageen-induced thermal hyperalgesia in rats. Eur J Pharmacol 1994;255: 123-129.
Eisenberg E, Vos BP, Strassman AM. The NMDA antagonist memantine blocks pain behavior in a rat model of formalin-induced facial pain. Pain 1993;54: 301-307.
First MB, Spitzer RL, Gibbon M, Williams JBW (1995): Structured clinical interview for DSM-IV Axis I Disorders - Patient Edition (SCID-I/P, Version 2.0). Biometrics Research Dept., New York State Psychiatric Institute
Fischer PA, Jacobi P, Schneider E et al. Die Wirkung intravenöser Gaben von Memantin bei Parkinson-Kranken. [The effects of intravenous administration of memantine in Parkinsonian patients]. Arzneim-Forsch/Drug Res 1977;27(II): 1487-1489.
Fleischhacker WW, Buchgeher A, Schubert H. Memantine in the treatment of senile dementia of the Alzheimer type. Prog Neuro -Psychopharmacol & Biol Psychiat 1986;10: 87-93.
Friston KJ, Holmes A, Poline J-B, et al. Detecting activations in PET and fMRI: Levels of inference and power. NeuroImage 1996;40: 223-235.
Goldberg JF, Harrow M, Leon AC. Lithium treatment of bipolar affective disorders under naturalistic followup conditions. Psychopharmacol Bull. 1996;32(1); 47-54.
Croodwin FK and Jamison KR. Manic-Depressive Illness. New York: Oxford University Press, 1990.
Görtelmeyer R and Erbler H. Memantine in the treatment of mild to moderate dementia syndrome. Arzneim-Forsch/Drug Res 1992;42: 904-913.
Greenamyre JT. Pharmacological pallidotomy with glutamate antagonists? Ann Neurol 1996;39: 557-558.
Greenberg PE, Sisitsky T, Kessler RC, et al. The economic burden of anxiety disorders in the 1990s. J Clin Psychiatry. 1999 Jul; 60:427-35.
Grossman W and Schütz W. Memantin und neurogene Blasenstörungen im Rhamen spatischer Zustandsbilder. [Memantine and Neurogenic bladder dysfunction in the frame work of the spastic clinical picture]. Arzneim-Forsch/Drug Res 1982;32: 1273-1276.
Hamilton M. A rating scale for depression. J Neurol Neurosurg Psychiatry. 1960; 23: 56-62
Hauang NU, Layer RT, Skolnick P. Is an adaptation of NMDA receptors an obligatory step in antidepressant action? In: Skoknick, P, Editor, 1997. Antidepressants: New Pharmacological Strategies Humana, Totowa, NJ, pp. 125-144.
Heimer L and Alheid GF (1991): Piecing together the puzzle of basal forebrain anatomy. In Napier TC, Kalivas PW, Hanin I (eds), The Basal Forebrain, New York, Plenum Press, pp. 1-42
Heresco-Levy U and Javitt DC. The role of N-methyl-D-aspartate (NMDA) receptor-mediated neurotransmission in the pathophysiology and therapeutics of psychiatric syndromes. Eur Neuropsychopharmacol 1988;8: 141-152.
Hirayasu Y, Shenton ME, Salisbury DF, et al, Subgenual cingulate cortex volume in first-episode psychosis. Am J Psychiatry 1999;156: 1091-1093.
Katona C, Abou-Saleh M, Harrison D, et al. Placebo-controlled trial of lithium augmentation of fluoxetine and lofepramine. Br J Psychiatry 1995;166: 80-86.
Kim JS, Schmid-Burgk W, Claus D, et al. Increased serum glutamate in depressed patients. Arch Psychiatrie Nervenkrankheiten 1982;232: 299-304.
Klockgether T and Turski L. Excitatory amino acids and the basal ganglia: implications for the therapy of Parkinson's disease. Trends Neurosci 1989;12: 285-286.
Klockgether T and Turski L. NMDA antagonist potentiate antiparkinsonian actins of L-dopa in monoamine depleted rats. Ann Neurol 1990;28: 539-546.
Kornhuber J, Bormann J, Hübers M, et al. Effects of 1-amine-adamantanes at the MK-801 binding site of the NMDA receptor gated ion channel: a human postmortem brain study, Eur J Pharmacol (Mod Pharmacol Sect) 1991;206: 297-300.
Kornhuber J, Bormann J, Retz W, et al. Memantine displaces [H3] MK-801 binding at therapeutic concentrations in post mortem human frontal cortex. Eur J Pharmacol 1989;166:589-590.
Kornhuber J, Weller M, Scchoppmeyer K, et al. Amantidine with memantine are NMDA receptor antagonists with neuroprotective properties. J Neural Transm Suppl 1994;43: 91-104.
Kornhuber J and Quack G. Cerebrospinal fluid and serum concentrations of the N-methy-D-aspartate (NMDA) receptor antagonist memantine in man. Neurosci Letters 1995;195: 137-139.
Laurenza A, Asnis G, Beaman M, Hudson J, Khan A, Londborg P, Monaghan E, Rudd D. A Double-Blind, Placebo-Controlled Study Supporting the Efficacy of Lamotrigine (Lamictal®) in Unipolar Depression, Bipolar Disorders 1999; 1(S1): 39-40.
Leon AC. Placebo protects subjects from nonresponse. A paradox of power. Arch Gen Psychiatry 2000; 57: 329-330
Leskow P. Therapie zentral bedingter Bewegungsstorungen: Multicenterstudie mit memantine. {The treatment of centrally determined movement disorders, Multicenter Study with memantine}. Therapiewoche 1987; 37; 4843-4845.
Levine J, Panchalingam K, Rapoport A, et al. Increased cerebrospinal fluid glutamine levels in depressed patients. Biol Psychiatry 2000;47: 586-593,
Lipton SA. Prospects for clinically-tolerated NMDA antagonists: open-channel blockers and alternative redox states of nitric oxide. Trends Neurosci 1993; 16: 527-532.
Lipton SA, Rosenberg RA. Mechanisms of disease: Excitatory amino acids as a final common pathway in neurologic disorders. N Engl J Med 1994;330: 613-622.
Maes M, Verkerk R, Vandoolaeghe E, et al. Serum levels of excitatory amino acids, serine, glycine, histidine, threonine, taurine, alanine and arginine in treatment resistant depression: modulation by treatment with antidepressants and prediction of clinical responsivity. Acta Psychiatr Scand. 1998 Apr;97(4): 302-8.
Maj J. Effects of memantine on the central neurotransmitter systemCA summary of the results. Arzneim Forsch/Drug Res 1982;32: 1236-1273,
Manji HK, Moore GJ, Chen G. Lithium at 50: have the neuroprotective effects of this unique cation been overlooked? Biol Psychiatry 1999;46(7): 929-40
Manji HK, Moore GJ, Chen G. Lithium up-regulates the cytoprotective protein Bcl-2 in the CNS in vivo: a role for neurotrophic and neuroprotective effects in manic depressive illness. J Clin Psychiatry 2000;61 Suppl 9: 82-96
Mathis P, Schmitt L, Benatia M, et al. Plasma amino acid disturbances and depression. Encephale 1988;14: 77-82.
Mauri MC, Ferrara A, Boscati L, et al. Plasma and platelet amino acid concentrations in patients affected by major depression and under fluvoxamine treatment. Neuropsychobiology. 1998;37(3):124-9.
McEwen BS and Sapolsky RM, Stress and cognitive function. Curr Opin Neurobiol. 1995 Apr;5(2): 205-16.
Mehta AK and Ticku. Role of N-methyl-D-aspartate (NMDA) receptors in experimental catalepsy in rats. Life Sci 1990;46: 37-42.
Meltzer CC, Kinahan PE, Greer PJ, et al. Comparative evaluation of MR-based partial volume correction schemes for PET. J Nucl Med 1999;40: 2053-2065
Merello M, Nouzeilles MI, Cammarota A, et al. Effect of memantine (NMDA antagonist) on Parkinson's Disease: A double-blind crossover randomized study. Clin Neuropharmacol 1999;22: 273-276.
Miltner FO, [Valve of symptomatic therapy with memantine in cerebral coma. II. Development of stretch synergisms in coma with brain stem symptoms]. Arzneimittelforschung. 1982;32(10): 1271-3.
Montgomery SA and Asberg M. A new depression scale designed to be sensitive to change. Br J Psychiatry 1979;134: 382-389.
Moore GJ, Bebchuk JM, Hasanat K, et al. Lithium increases N-acetyl-aspartate in the human brain: in vivo evidence in support of bcl-2's neurotrophic effects? Biol Psychiatry 2000a/48: 1-8.
Moore GJ, Bebchuk JM, Wilds IB, et al. Lithium-induced increase in human brain gray matter. Lancet 2000b;356: 1241-2*.*
Mundinger F and Milios E. [Experiences with memantine in the treatment of severe spastic and extrapyramidal movement disorders in combination with stereotaxic surgery]. Nervenarzt. 1985 Feb;56(2): 106-9.
Murray CJ and Lopez AD. Global mortality, disability, and the contribution of risk factors: Global Burden of Disease Study Lancet. 1997;349: 1436-42,
Musselman DL, Evans DL, Nemeroff CB. The relationship of depression to cardiovascular disease: epidemiology, biology, and treatment Arch Gen Psychiatry. 1998;55:580-92.
Nakamura S. Antidepressants induce regeneration of catecholaminergic axon terminals in the rat cerebral cortex. NeurosciLett 1990; 111: 64-8*.*
Nibuya M, Morinobu S, Duman RS. Regulation of BDNF and trkH mRNA in rat brain by chronic electroconvulsive seizure and antidepressant drug treatments. J Neurosci. 1995 Nov;15 (11): 7539-47.
Nibuya M, Nestler EJ, Duman RS. Chronic antidepressant administration increases the expression of cAMP response element binding protein (CREB) in rat hippocampus. J Neurosci, 1996 Apr 1;16(7): 2365-72.
Nierenberg AA: Methodological problems in treatment resistant depression research. Psychopharmacol Bull. 1994;190;26: 461-464.
Nijolajsen L, Gotrrup H, Kristensen AGD, et al, Memantine (a N-methyl-D-aspartate receptor antagonist) in the treatment of neuropathic pain after amputation or surgery: A randomized, double-blind, cross-over study. Anesth Analg 2000;91: 960-966.
Nikolajsen L, Hansen CL, Nielsen J, et al. The effect of ketamine on phanthom pain: a central neuropathic disorder maintained by peripheral input. Pain 1996;67: 69-77.
Nowak G, Ordway GA, Paul IA. Alteration in the N-methyl-D-aspartate (NMDA) receptor complex in the frontal cortex of suicide victims. Brain Res 1995;675: 157-164.
Nowak G, Trullas R, Layer RT, et al. Adaptive changes in the N-methyl-D-aspartate receptor complex after chronic treatment with imipramine and 1-aminocyclopropanecarboxylic acid. J Pharmacol Exp Ther 1993;265: 1380-1386.
Ongur D, Drevets WC, Price JL. Glial reduction m the subgenual prefrontal cortex in mood disorders. Proc Natl Acad Sci U SA 1998; 95: 13290-13295.
Ossowska K, Pietraszek M, Wardas J, et al. The role of glutamate receptors in antipsychotic drug action. Amino Acids. 2000;19(1): 87-94.
Pantev M, Ritter R, Gortelmeyer R Clinical and behavioural evaluation in long-term care patients with mild to moderate dementia under memantine treatment. Zeitschrift für Gerontopsychologie und Psychiatrie 1993; 6:103-117.
Parker G and Hadzi-Pavlovic D (Eds) (1996): Melancholia: A Disorder of Movement and Mood. New York: Cambridge University Press,
Paul IA, Nowak G, Layer RT, et al. Adaptation of the N-methyl-D-aspartate receptor complex following chronic antidepressant treatments. J Pharmacol Exp Ther 1994;269:95-102.
Paul IA. NMDA receptors and affective disorders. In Skolnick, P. Editor. Antidepressants: New Pharmacological Strategies Humana, Totowa, NJ, 1997, pp. 145-158.
Puigdemont D, Perez V, Perez-Blanco J et al. A follow-up study of resistant major depression. In: Abstracts of the X World Congress of Psychiatry; August 23-28, 1996; Madrid, Spain; 1996;2: 3 36-337.
Rabey JM, Nissipeanu P, Korczyn AD. Efficacy of memantine, an NMDA receptor antagonist in the treatment of Parkinson's Disease. J Neural Transm 1992;4: 277-282.
Rajkowska G. Postmortem studies in mood disorders indicate altered numbers of neurons and glial cells. Biol Psychiatry 2000;48: 766-77*.*
Rajkowska G, Miguel-Hidalgo JJ, Wei J, et al. Morphometric evidence for neuronal and glial prefrontal cell pathology in major depression. Biol Psychiatry 1999;45:1085-98.
Rohde H. [Results of clinical trials of the antispasmodic compound memantine]. Fortschr Med. 1982 Nov 18;100(43): 2023-6.
Rosenberg DR, MacMaster FP, Keshavan MS, et al. Decrease in caudate glutamatergic concentrations in pediatric obsessive-compulsive disorder patients taking paroxetine. J Am Acad Child Adolesc Psychiatry. 2000 Sep;39(9): 1096-103.
Rusinek H and Chandra R. Brain tissue volume measurement from magnetic resonance imaging. Invest Radiol 1993;28: 890-895.
Sapolsky RM. The possibility of neurotoxicity in the hippocampus in major depression: a primer on neuron death. Biol Psychiatry 2000;48(8): 755-65
Schmidt WJ, Bubser M, Hauber W. Excitatory amino acids and Parkinson's disease. Trends Neurosci 1990;13: 46-47.
Schneider E, Fischer PA, Clemens R, et al. Wirkungen oraler Memantin-Gaben auf die Parkinson-Symptomatik. {Effects of oral memantine administration on Parkinson symptoms; Results of a placebo-controlled multicenter study}. Dtsch Med Wschr 1984;109: 987-990.
Schulz R, Beach SR, Ives DG, Martire LM, Ariyo AA, Kop WJ. Association between depression and mortality in older adults: the Cardiovascular Health Study, Arch Intern Med. 2000 Jun 26;160(12): 1761-8.
Sheline YI, Sanghavi M, Mintun MA, et al. Depression duration but not age predicts hippocampal volume loss in medically health women with recurrent major depression. J Neurosci 1999;19: 5034-43.
Sheline YI, Wang PW, Gado MH, et al. Hippocampal atrophy in recurrent major depression. Proc Natl Acad Sci USA 1994;93: 3908-13.
Skolnick P. Antidepressants for the new millennium. Eur J Pharmacol. 1999 Jun 30;375(1-3): 31-40.
Thase ME and Rush AJ. Treatment-resistant depression. In; Bloom FE, Kupfer DJ, eds. Psychopharmacology: The Fourth Generation of Progress,.New York, NY: Raven Press; 1995: 1081-1098.
Tohen M, Hennen J, Zarate CM Jr, Baldessarini RJ, Strakowski SM, Stoll AL, Faedda. GL, Suppes T, Gebre-Medhin P, Cohen BM. Two-year syndromal and functional recovery in 219 cases of first-episode major affective disorder with psychotic features. Am J psychiatry. 2000 Feb;157(2): 220-8.
Tsai G and Coyle JT. N-acetylaspartate in neuropsychiatric disorders. Prog Neurobiol 1995;46: 531-40.
Walder DJ, Walker EF, Lewine RJ. Cognitive functioning, cortisol release, and symptom severity in patients with schizophrenia. Biol Psychiatry 2000;48: 1121-1132,
Weseman W, Sturn G, Funfgeld EW. Distribution of metabolism of the potential anti-Parkinson drug memantine in the human. J Neural Transm Suppl 1980;143-148.
West J, Fitzpatrick J, Wang MY et al. Comparison and evaluation of retrospective intermodality brain image registration techniques. J Comp Assist Tomogr 1997;21(4): 554-66
Winsberg ME, Sachs N, Tate DL, et al. Decreased dorsolateral prefrontal N-acetyl aspartate in bipolar disorder. Biol Psychiatry 2000;47: 415-81.
Wong JCH, Studholme C, Hawkes DJ, et al. Evaluation of the limits of visual detection of image misregistration in a brain fluorine-18 fluorodeoxyglucose PET-MRI study. Eur J Nucl Med 1997;24 (6): 642-50
Woods RP, Masotho J, Cherry SR (1993): MRI-PET registration with automated algorithm. J Comput Assist Tomogr 17: 536-546
Wu JC, Gillin JC, Buchsbaum MS, et al. Effect of sleep deprivation on brain metabolism of depressed patients. Am J Psychiatry 1992;149: 538-543.
Wyatt RJ and Henter I. An economic evaluation of manic-depressive illness-1991. Soc Psychiatry Psychiatr Epidemiol. 1995;30: 213-9.
Young RC, Biggs JR, Ziegler VE, et al. A rating scale for mania: reliability, validity and sensitivity. Br J Psychiatry 1978;133: 429-435

## Claims

1. A method for treating neuropsychiatric disorders comprising administering to a human patient suffering from a neuropsychiatric disorder, an effective amount of an NMDA receptor antagonist compound to modulate glutamatergic neurotransmission by NMDA receptors in the human patient, thereby treating the neuropsychiatric disorder.

2. The method of claim 1, wherein the neuropsychiatric disorder is major depression.

3. The method of claim 1, wherein the neuropsychiatric disorder is bipolar disorder.

4. The method of claim 1, wherein the neuropsychiatric disorder is anxiety,

5. The method of claim 1, wherein the neuropsychiatric disorder is selected from the group consisting of: drug addiction, drug dependency, drug withdrawal, and drug tolerance.

6. The method of claim 1, wherein excessive glutamatergic neurotransmission is modulated, thereby mediating an excitotoxic effect of glutamate on neurons.

7. The method of claim 6, wherein mediating the excititoxic effect of glutamate on neurons provides a neuroprotective effect.

8. The method of claim 1, wherein the NMDA receptor antagonist compound modulates NMDA receptor activity in a glutamatergic cortico-striatal or a subthallamicopalladial pathway.

9. The method of claim 8, wherein the NMDA receptor antagonist compound modulates activity in the glutamatergic cortico-striatal or the subthallamicopalladial pathways independent of dopamine or norepinephrine release.

10. The method of claim 1, wherein the NMDA receptor antagonist compound comprises memantine, nitromemantine, its enantiomers, or a pharmaceutically acceptable salt thereof and the compound is administered to the human patient from a dosage of 0.1 mg/day to 100 mg/day.

11. The method of claim 10, wherein the NMDA receptor antagonist compound is administered to the human patient from a dosage of 5 mg/day to 80 mg/day.

12. The method of claim 10, wherein the NMDA receptor antagonist compound is administered to the human patient from a dosage of 10 mg/day to 35 mg/day.

13. The method of claim 10, wherein the patient suffering from the neuropsychiatric disorder is thereby provided with a therapeutic effect.

14. A method of using a NMDA receptor antagonist compound to modulate glutamatergic neurotransmission in a human patient comprising administering to a patient suffering from a neuropsychiatric disorder, an effective amount of a NMDA receptor antagonist compound to antagonize NMDA receptors in the human patient, thereby modulating glutamatergic neurotransmission by the NMDA receptors and thereby treating the neuropsychiatric, disorder.

15. The method of claim 14, wherein the neuropsychiatric disorder is major depression.

16. The method of claim 14, wherein the neuropsychiatric disorder is bipolar disorder.

17. The method of claim 14, wherein the neuropsychiatric disorder is anxiety.

18. The method of claim 14, wherein the neuropsychiatric disorder is selected from the group consisting of drug addiction, drug dependency, drug withdrawal, and drug tolerance.

19. The method of claim 14, wherein antagonizing NMDA receptors in the human patient thereby mediates an excitotoxic effect of glutamate on neurons.

20. The method of claim 19, wherein mediating the excititoxic effect of glutamate on neurons provides a neuroprotective effect.

21. The method of claim 14, wherein the NMDA receptor antagonist compound modulates NMDA receptor activity in a glutamatergic cortico-striatal or a subthallamicopalladial pathway.

22. The method of claim 21, wherein the NMDA receptor antagonist compound modulates activity in the glutamatergic cortico-striatal or the subthallamicopalladial pathway independent of dopamime or norepinephrine release.

23. The method of claim 14, wherein the NMDA receptor antagonist compound comprises memantine, nitromemantine, its enantiomers, or a pharmaceutically acceptable salt thereof, and the compound is administered to the human patient from a dosage of 0.1 mg/day to 100 mg/day.

24. The method of claim 14, wherein the NMDA, receptor antagonist compound is administered to the human patient from a dosage of 5 mg/day to 80 mg/day.

25. The method of claim 14, wherein the NMDA receptor antagonist compound is administered to the human patient from a dosage of 10 mg/day to 35 mg/day.

26. The method of claim 14, wherein antagonizing NMDA receptors in the human patient thereby provides a therapeutic effect to the human patient suffereing from the neuropsychiatric disorder.

27. A method of using a NMDA receptor antagonist compound to modulate glutamatergic neurotransmission in a human patient comprising administering to a patient suffering from a neuropsychiatric disorder, an effective amount of NMDA receptor antagonist compound to antagonize the PCP or MK-801 binding site of NMDA receptors in the human patient, thereby modulating excessive glutamatergic neurotransmission by the NMDA receptors, thereby providing the human patient a neuroprotective effect and a neurotropic effect, and thereby treating the neuropsychiatric disorder.

28. The method of claim 27, wherein the NMDA receptor antagonist compound comprises memantine, nitromomantine, its enantiomers, or a pharmaceutically acceptable salt thereof, and the compound is administered to the human patient from a dosage of 0.1 mg/day to 100 mg/day.

29. The method of claim 27, wherein the NMDA receptor antagonist compound is administered to the human patient from a dosage of 5 mg/day to 80 mg/day.

30. The method of claim 27, wherein the NMDA receptor antagonist compound is administered to the human patient from a dosage of 10 mg/day to 35 mg/day.

31. The method of claim 27, wherein the neuropsychiatric disorder is selected from the group consisting of: major depressive disorder, bipolar disorder, anxiety, drug addiction, drug dependency, drug withdrawal, and drag tolerance,

32. The use of an NMDA receptor antagonist compound in the manufacture of a medicament to modulate excessive glutamatergic neurotransmission by NMDA receptors for treatment of a human patient suffering from a neuropsychiatric disorder.

33. The use of an NMDA receptor antagonist compound as defined in claim 32 to antagonize the PCP or MK-801 binding site of NMDA receptors in the human patient.

34. The use of an NMDA receptor antagonist compound as defined in claim 33 to modulate excessive glutamatergic neurotransmission by the NMDA receptors, thereby providing the human patient a neuroprotective effect and a neurotropic effect, and thereby treating the neuropsychiatric disorder.

35. The use of an NMDA receptor antagonist compound as defined in claim 32, wherein the medicament provides the human patient with a dosage of the NMDA receptor antagonist compound from 0.1 mg/day to 100 mg/day.

36. The use of an NMDA receptor antagonist compound as defined in claim 32, wherein the medicament provides the human patient with a dosage of the NMDA receptor antagonist compound from 5 mg/day to 80 rug/day.

37. The use of an NMDA receptor antagonist compound as defined in claim 32, wherein the medicament provides the human patient with a dosage of the NMDA receptor antagonist compound from 10 mg/day to 35 mg/day.
